# FASCICULE DE BREVET EUROPEEN

(11) **EP 2 496 278 B1**
(45) Date de publication et mention de la délivrance du brevet: **09.09.2015**
(21) Numéro de dépôt: 10785098.4
(22) Date de dépôt: 29.10.2010
(51) Int. Cl.: A61B 5/00, A61M 5/14, A61B 5/155, A61B 5/145, A61B 5/153, A61M 1/02, A61M 5/168, A61M 5/172, A61B 5/157

(54) **SYSTÈME DE PERFUSION SÉCURISÉ**
SICHERES PERFUSIONSSYSTEM
SECURE PERFUSION SYSTEM

(30) Priorité: 05.11.2009 FR 0905329
(43) Date de publication de la demande: 12.09.2012
(73) Titulaire: Centre National de la Recherche Scientifique, 75016 Paris (FR); Centre Hospitalier Universitaire de Besançon, 25030 Besançon Cedex (FR); Université de Franche-Comté, 25000 Besançon (FR); Etablissement Français du Sang, 93218 La Plaine Saint Denis Cedex (FR)
(72) Inventeur: PAZART, Lionel, Henri, 25000 Besançon (FR); WACOGNE, Bruno, François, Marcel, 70190 Traitiefontaine (FR); PIERALLI, Christian, Gérard, Daniel, 25000 Besançon (FR); BOIREAU, Wilfrid, 25680 Mondon (FR); MOREL, Pascal, Charles, Serge, 25000 Besançon (FR)
(74) Mandataire: Reboussin, Yohann Mickaël Noël
(86) Numéro de dépôt international: PCT/FR2010/000714
(87) Numéro de publication internationale: WO 2011/055031

(56) Documents cités:
- WO-A1-99/56630
- WO-A2-2006/088771
- WO-A2-2007/033025
- US-A- 4 573 968
- US-A- 4 900 321

## Description

L'invention se rapporte à un système de perfusion sécurisé, en particulier de transfusion sanguine, et à un procédé de mise en oeuvre.

Après que le médecin ait diagnostiqué une situation médicale donnée (maladie, accident, hémorragie, etc.), il peut être nécessaire de la soigner en administrant au patient un produit adapté : un médicament, du sang, etc... L'invention se rapporte à une administration par injection, en particulier par une perfusion;

De manière connue, une perfusion sur une veine périphérique est mise en place de la manière suivante.

Après avoir repéré une veine périphérique, on pique la peau du patient à l'aide d'une aiguille enrobée d'un cathéter (un tel dispositif est connu sous la marque Cathlon®). Une fois l'aiguille positionnée dans une veine, on pousse l'extrémité distale du cathéter dans la veine, on retire l'aiguille, et on fixe l'extrémité proximale du cathéter sur la peau du patient par un pansement stérile. Différents diamètres de cathéter existent, et sont généralement exprimés en Gauge (G). Le diamètre du cathéter utilisé dépend de l'état du patient, de l'opération à réaliser (prélèvement ou injection) et de l'âge du patient. Par exemple, on choisira un cathéter de 22 à 24 Gauge pour les nouveau-nés, de 22 Gauge pour les enfants de 1 mois à 3 ans, de 20 Gauge pour les enfants plus grands. Pour les adultes, on pourra choisir des cathéters de 18 Gauge (soit un diamètre externe de 1,1 mm à 1,3 mm) jusqu'à 14 Gauge (soit un diamètre externe de 1,8 à 2,2 mm).

Une table de correspondance entre l'unité « Gauge » et le système métrique est donné ci-après :

| Gauge (diamètre interne de l'aiguille) | Diamètre externe (mm) |
|---|---|
| 24 G | 0,6 à 0,7 |
| 22 G | 0,7 à 0,9 |
| 20 G | 0,9 à 1,1 |
| 18 G | 1,1 à 1,3 |
| 16 G | 1,5 à 1,8 |
| 14 G | 1,8 à 2,2 |

Le cathéter porte, à son extrémité proximale, un dispositif de connexion, par exemple de type luer-lock, à l'extrémité distale d'une tubulure de perfusion. Ce dispositif de connexion peut être tel que lorsque le cathéter n'est pas connecté, son extrémité proximale est fermée et le sang ne peut pas sortir.

En général, après la connexion au cathéter, on réalise une boucle de sécurité avec la tubulure que l'on fixe par un adhésif sur la peau du patient. Cette boucle de sécurité évite l'arrachage immédiat du cathéter en cas de traction sur la tubulure de perfusion.

L'extrémité proximale de la tubulure est en communication fluidique avec un vase d'expansion connecté à une poche rigide ou souple de produit de perfusion fixée à une perche. Cette dernière doit être suffisamment haute par rapport au cathéter du patient pour que le produit de perfusion s'écoule par simple gravité vers le cathéter puis le système vasculaire du patient.

La tubulure comprend, de préférence, une molette de réglage du débit de perfusion, ou autre système de régulation.

Avant de faire circuler le produit par la perfusion, il est essentiel d'effectuer un contrôle ultime de compatibilité entre le traitement préalablement prescrit par le médecin et le traitement que l'on s'apprête à donner au patient. Ce contrôle permet de vérifier que le produit correspond bien à la situation médicale diagnostiquée et/ou qu'il sera bien toléré par le patient.

Par exemple, pour la transfusion sanguine, lorsqu'un médecin a prescrit une transfusion pour un patient de groupe sanguin donné, l'infirmière doit s'assurer que les poches de sang dont elle dispose sont compatibles avec le groupe sanguin du patient car il peut exister des erreurs d'attribution des poches de sang ou d'identification du patient ou des poches de sang.

Classiquement, l'infirmière utilise un papier cartonné imprégné de réactif anti-A et anti-B sur lequel elle dépose du sang du patient (obtenu par une piqûre au bout du doigt ou dans une veine) et du sang issu d'un échantillon de la poche de transfusion. A cette fin, les poches de transfusion présentent un réservoir principal pour la transfusion, et des réservoirs secondaires d'échantillonnage, séparables du réservoir principal.

L'infirmière apprécie alors la présence d'agglutinats sur le papier et compare les réactions obtenues avec le sang du patient et avec le sang de l'échantillon. Elle doit alors appliquer les règles de compatibilité qu'elle a apprise pour interpréter les résultats du test. Cette interprétation peut être délicate, notamment en présence d'antigènes faibles ou dans certaines pathologies.

Ainsi, on s'est aperçu qu'il existe encore de nombreuses erreurs dues à la fatigue, à une situation d'urgence ou à une inattention, lors de l'interprétation de ces tests, mais également lors de l'installation de la poche de transfusion et, plus généralement, de la deuxième perfusion.

En particulier, on s'est aperçu qu'encore trop souvent le produit de la deuxième perfusion n'est pas celui qui avait été prescrit par le médecin.

Ces erreurs peuvent simplement ralentir la guérison du patient, par exemple lorsque le dosage du produit perfusé est inférieur à celui qui est prescrit par le médecin pour guérir le patient. Elles peuvent également entraîner la mort du patient, par exemple lorsque du sang transfusé est incompatible avec le groupe sanguin (A, B, AB ou O) du patient, ou lorsque le patient est allergique à l'antibiotique perfusé alors qu'il n'était pas allergique à l'antibiotique prescrit initialement.

Il peut également survenir des erreurs de manipulation dangereuses pour le personnel soignant, s'il y a une exposition directe au sang du patient.

Pour palier ce problème, les solutions actuelles visent à s'assurer de la concordance des informations inscrites sur la poche de transfusion et des informations inscrites sur un support porté par le patient. Ces technologies consistent, principalement, en des systèmes de contrôle à code-barres ou puce RFID. Cependant, il existe encore des erreurs de manipulation notamment en raison d'erreurs d'étiquetage des poches de perfusion ou d'identification des patients.

Un premier objectif de la présente invention est donc de proposer un système de perfusion permettant de réaliser, de manière simple, efficace et économe en énergie, un contrôle ultime de compatibilité d'un traitement, préalablement choisi par un médecin, avec le patient et/ou la situation médicale préalablement diagnostiquée par un médecin.

Pour cela, l'invention propose de réaliser un système de perfusion comprenant un contrôle ultime sur la même ligne de perfusion connectée au patient.

Par ailleurs, lorsqu'un patient (humain ou animal) est perfusé, par exemple avec un produit thérapeutique (solution saline, solution antibiotique, etc.), il peut être nécessaire de lui faire une deuxième perfusion d'un autre produit, tel que du sang, après que le médecin ait diagnostiqué une situation médicale donnée (maladie, accident, hémorragie, etc.). Dans l'exemple décrit, il s'agit d'un besoin de sang.

Dans ce cas, une personne autorisée (médecin, infirmière ou vétérinaire) doit installer une deuxième ligne de perfusion, spécifique pour la transfusion de sang.

Classiquement pour une voie sur une veine périphérique, la deuxième perfusion est installée sur le bras controlatéral. Le patient subit donc une première piqûre pour la pose de la première perfusion, puis une deuxième piqûre pour la pose de la deuxième perfusion.

Dans le cadre la présente invention, un prélèvement de liquide corporel est effectué pour réaliser un contrôle ultime avant d'injecter le produit de la deuxième perfusion. Le patient devrait donc subir une troisième piqûre pour le prélèvement.

Une autre possibilité consiste à mettre en place une tubulure équipée d'un robinet à trois positions afin d'y connecter la tubulure de la deuxième perfusion sans nécessiter de piquer à nouveau le patient. Puis, dans cet exemple, le patient devra subir une deuxième piqûre pour le prélèvement.

Pour éviter de faire une deuxième piqûre à un patient déjà perfusé, le document WO2006/088771 propose de munir une perfusion classique d'une pompe réversible associée à un moyen de commande. Ce dernier est conçu pour interrompre, par intermittence, le fonctionnement de la pompe à perfusion dans le sens avant (c'est-à-dire depuis la poche de produit de perfusion vers le patient), afin de faire fonctionner la pompe dans le sens arrière (c'est-à-dire depuis le patient vers un circuit de prélèvement). De cette manière, il est possible de prélever du sang du patient par le cathéter de perfusion.

Cependant, ce dispositif nécessite un réglage minutieux de la pompe et un circuit fluidique complexe comprenant de multiples vannes.

En outre, ce système peut être bruyant (en raison du fonctionnement de la pompe) et nécessite une source d'énergie pour le prélèvement de sang et l'injection du produit de perfusion.

Un autre objectif de l'invention est donc de proposer un dispositif simple, efficace et économe en énergie, permettant de réaliser de manière aisée un prélèvement de liquide corporel en évitant un nouveau geste intrusif dans le corps du patient, et en limitant la nécessité d'une purge préalable au prélèvement.

Le positionnement du cathéter de perfusion dans le corps du patient ne fait pas partie de la présente invention. Au contraire, l'invention permet de profiter d'une perfusion déjà installée chez un patient pour effectuer un prélèvement de liquide corporel, tel que du sang, et éviter une nouvelle piqûre dans le corps du patient.

A cette fin, l'invention a pour objet un système de perfusion sécurisé d'un liquide corporel, comprenant :
- un circuit fluidique d'une perfusion comprenant un cathéter de perfusion, une tubulure de perfusion et un réservoir de produit de perfusion déterminé à perfuser à un patient ;
- un moyen de prélèvement d'un liquide corporel d'un patient,
- un réservoir de prélèvement muni d'un moyen de liaison fluidique avec le moyen de prélèvement,
- un moyen d'analyse et de comparaison du liquide corporel prélevé et d'un échantillon de produit déterminé,
- un moyen de liaison fluidique entre le moyen d'analyse et le réservoir de prélèvement ;
- un moyen de commande d'écoulement du produit de perfusion dans la tubulure, le moyen d'analyse et de comparaison étant apte à transmettre au moyen de commande d'écoulement une information de comparaison du liquide corporel prélevé et de l'échantillon de produit déterminé.

Selon d'autres modes de réalisation :
- le moyen d'analyse peut être apte à contrôler le moyen de commande d'écoulement pour qu'il bloque l'écoulement du produit de perfusion si l'information de comparaison indique que le liquide corporel et le produit de perfusion ne sont pas compatibles, et pour qu'il autorise l'écoulement du produit de perfusion si l'information de comparaison indique que le liquide corporel et le produit de perfusion sont compatibles ;
- le système de perfusion sécurisé peut comprendre un moyen d'affichage de l'information de comparaison du liquide corporel prélevé et de l'échantillon de produit déterminé ;
- le moyen d'analyse peut être apte à contrôler le moyen de commande d'écoulement pour qu'il génère automatiquement l'écoulement du produit de perfusion si le liquide corporel et le produit de perfusion sont compatibles, et pour qu'il ne génère pas l'écoulement du produit de perfusion si le liquide corporel et le produit de perfusion ne sont pas compatibles ;
- le moyen d'analyse peut comprendre une chambre de réaction et un moyen de détection ;
- le moyen de prélèvement peut comprendre :
   - un canal de prélèvement comprenant une extrémité proximale destinée à être reliée au réservoir de prélèvement, et une extrémité distale, et
   - un dispositif de prélèvement destiné à être incorporé dans le circuit fluidique d'une perfusion, et comprenant une structure tubulaire de connexion au circuit fluidique de la perfusion, munie :
      o d'une zone d'intubation, en utilisation, d'un canal de prélèvement comprenant une extrémité distale ; et
      o d'un moyen de maintien, en utilisation, dans la structure tubulaire, de la partie du canal de prélèvement comprenant une extrémité distale, de sorte que, en utilisation, ladite extrémité distale soit dirigée vers le cathéter de perfusion, dans le sens d'écoulement du produit de perfusion, depuis un réservoir de produit de perfusion vers le cathéter de perfusion ;
- la zone d'intubation peut être agencée pour que, en utilisation, elle soit disposée à une distance verticale maximale déterminée de l'extrémité proximale du cathéter.
- la distance verticale maximale déterminée peut être comprise entre 0 cm et 50 cm ;
- la partie comprenant l'extrémité distale du canal de prélèvement peut être fixée dans la structure tubulaire, et une partie comprenant une extrémité proximale du canal de prélèvement débouche hors la structure tubulaire au niveau de la zone d'intubation ;
- la zone d'intubation peut comprendre un moyen d'insertion adapté pour permettre, en utilisation, l'insertion, dans la structure tubulaire, de la partie du canal de prélèvement comprenant l'extrémité distale ;
- le moyen d'insertion peut être choisi parmi :
   - une membrane en un matériau étanche conservant son étanchéité après avoir été percé ; et
   - un connecteur étanche.
- le matériau étanche conservant son étanchéité après avoir été percé peut être choisi parmi un polymère de silicone, tel que le polydiméthylsiloxane (PDMS), du polyméthylmétacrylate (PMMA), du Polychlorure de vinyle (PVC), et du Tygon® ;
- le dispositif de prélèvement peut comprendre, en outre :
   - un moyen de connexion à une extrémité proximale du cathéter de la perfusion, et
   - un moyen de connexion à une extrémité distale de la tubulure de la perfusion.
- le canal de prélèvement peut présenter une longueur entre ses deux extrémités comprise entre 10 cm et 100 cm, de préférence entre 20 cm et 50 cm.
- le système de perfusion sécurisé peut comprendre un cathéter de perfusion comprenant :
   - une extrémité distale destinée à être insérée dans un patient ;
   - une extrémité proximale destinée à être connectée avec une extrémité distale de la tubulure de la perfusion ;
   - le dispositif de prélèvement arrangé entre l'extrémité distale et l'extrémité proximale, et comprenant une structure tubulaire de connexion au circuit fluidique de la perfusion, munie :
      o d'une zone d'intubation, en utilisation, d'un canal de prélèvement comprenant une extrémité distale ; et
      o d'un moyen de maintien, en utilisation, dans la structure tubulaire, de la partie du canal de prélèvement comprenant une extrémité distale, de sorte que, en utilisation, ladite extrémité distale soit dirigée vers le cathéter de perfusion, dans le sens d'écoulement du produit de perfusion, depuis un réservoir de produit de perfusion vers le cathéter de perfusion ;
- le système de perfusion sécurisé peut comprendre une tubulure de perfusion comprenant :
   - une extrémité proximale destinée à être connectée à un réservoir d'un produit de perfusion ;
   - une extrémité distale destinée à être connectée avec une extrémité proximale d'un cathéter de perfusion insérée dans un patient ;
   - le dispositif de prélèvement arrangé entre l'extrémité distale et l'extrémité proximale, et comprenant une structure tubulaire de connexion au circuit fluidique de la perfusion, munie :
      ∘ d'une zone d'intubation, en utilisation, d'un canal de prélèvement comprenant une extrémité distale ; et
      ∘ d'un moyen de maintien, en utilisation, dans la structure tubulaire, de la partie du canal de prélèvement comprenant une extrémité distale, de sorte que, en utilisation, ladite extrémité distale soit dirigée vers le cathéter de perfusion, dans le sens d'écoulement du produit de perfusion, depuis un réservoir de produit de perfusion vers le cathéter de perfusion ;
- la zone d'intubation peut être disposée à une distance verticale maximale déterminée de l'extrémité proximale du cathéter ; et/ou
- la distance verticale maximale déterminée peut être comprise entre 0 cm et 50 cm.

L'invention se rapporte également à un procédé de mise en oeuvre d'un système de perfusion sécurisé précédent, dont le moyen de prélèvement a été préalablement installé à un patient, comprenant les étapes suivantes :
- mettre en communication fluidique le moyen de prélèvement, le réservoir de prélèvement et le moyen d'analyse et de comparaison,
- amener le réservoir de prélèvement à une hauteur inférieure à celle du cathéter du patient, de telle sorte que le liquide corporel s'écoule dans le canal de prélèvement, vers le réservoir de prélèvement ;
- maintenir le réservoir de prélèvement à une hauteur inférieure à celle du cathéter du patient pendant une durée suffisante pour obtenir, dans le réservoir de prélèvement, un volume suffisant pour constituer un prélèvement de liquide corporel ;
- analyser et comparer le liquide corporel prélevé et l'échantillon de produit déterminé ;
- générer une information de comparaison du liquide corporel prélevé et de l'échantillon de produit déterminé ;
- transmettre l'information de comparaison à un moyen de commande d'écoulement du produit de perfusion ;
- bloquer l'écoulement du produit de perfusion si l'information de comparaison indique que le liquide corporel et le produit de perfusion ne sont pas compatibles, et autoriser l'écoulement du produit de perfusion si l'information de comparaison indique que le liquide corporel et le produit de perfusion sont compatibles.

Selon d'autres modes de réalisation :
- le procédé peut comprendre, en outre, une étape de d'affichage de l'information de comparaison du liquide corporel prélevé et de l'échantillon de produit déterminé.
- le procédé peut comprendre les étapes suivantes :
   - incorporer le dispositif de prélèvement dans un circuit fluidique d'une première perfusion préalablement installée sur un patient muni d'un cathéter de perfusion, dans une partie externe du circuit fluidique par rapport au corps du patient ;
   - maintenir, à l'intérieure de la structure tubulaire, la partie du canal de prélèvement comprenant l'extrémité distale, de sorte que ladite extrémité distale soit dirigée vers le cathéter de perfusion, dans le sens d'écoulement du produit de perfusion, depuis un réservoir de produit de perfusion vers le cathéter de perfusion ;
   - mettre en communication fluidique le canal de prélèvement, le réservoir de prélèvement et le moyen d'analyse et de comparaison,
   - amener le réservoir de prélèvement à une hauteur inférieure à celle du cathéter du patient, de telle sorte que le liquide corporel s'écoule dans le canal de prélèvement, à contre-courant par rapport au produit de perfusion, puis vers le réservoir de prélèvement ;
   - maintenir le réservoir de prélèvement à une hauteur inférieure à celle du cathéter du patient pendant une durée suffisante pour obtenir, dans le réservoir de prélèvement, un volume suffisant pour constituer un prélèvement de liquide corporel ;
   - analyser et comparer le liquide corporel prélevé et un échantillon de produit déterminé ;
   - générer une information de comparaison du liquide corporel prélevé et de l'échantillon de produit déterminé ;
   - transmettre l'information de comparaison à un moyen de commande d'écoulement du produit d'une deuxième perfusion ;
   - bloquer l'écoulement du produit de la deuxième perfusion si l'information de comparaison indique que le liquide corporel et le produit de la deuxième perfusion ne sont pas compatibles, et autoriser l'écoulement du produit de la deuxième perfusion si l'information de comparaison indique que le liquide corporel et le produit de la deuxième perfusion sont compatibles ;
- le dispositif de prélèvement peut être agencé pour que, en utilisation, la zone d"intubation soit disposée à une distance verticale maximale déterminée de l'extrémité proximale du cathéter ;
- la distance verticale maximale déterminée peut être comprise entre 0 cm et 50 cm ; et/ou
- l'extrémité distale du canal de prélèvement peut être agencée à une distance déterminée d'une extrémité proximale du cathéter de perfusion, ladite distance étant dite « distance d'abouchement », et comprise entre 0 cm et 20 cm, de préférence entre 0 cm et 3 cm.

D'autres caractéristiques de l'invention seront énoncées dans la description détaillée ci-après, faite en référence aux figures annexées qui représentent, respectivement :
- la figure 1, une vue schématique en plan d'un premier mode de réalisation d'un système de perfusion sécurisé selon l'invention ;
- la figure 2, une vue schématique en plan d'un deuxième mode de réalisation d'un système de perfusion sécurisé selon l'invention ;
- la figure 3, une vue schématique en plan d'un troisième mode de réalisation d'une utilisation particulière du dispositif de prélèvement selon l'invention.
- la figure 4, une vue schématique en plan d'un quatrième mode de réalisation d'un système de perfusion sécurisé selon l'invention, comprenant un dispositif de prélèvement évitant une deuxième piqûre au patient ;
- la figure 4a, un agrandissement partiel de la figure 4 illustrant, en détail, le dispositif de prélèvement selon l'invention ;
- la figure 5, une vue schématique en plan d'un cinquième mode de réalisation d'un système de perfusion sécurisé selon l'invention comprenant deux perfusions ;
- la figure 5a, un agrandissement partiel de la figure 5 illustrant, en détail, le dispositif de prélèvement selon l'invention ;
- la figure 6, une vue schématique en plan d'un sixième mode de réalisation d'un système de perfusion sécurisé selon l'invention comprenant deux perfusions ;
- la figure 6a, un agrandissement partiel de la figure 3 illustrant, en détail, le dispositif de prélèvement selon l'invention;
- la figure 7, une vue schématique en plan d'un septième mode de réalisation d'un système de perfusion sécurisé selon l'invention comprenant deux perfusions ;
- la figure 7a, un agrandissement partiel de la figure 7 illustrant, en détail, le dispositif de prélèvement selon l'invention ;
- les figures 8 et 9, des vues schématiques en plan de deux modes de réalisation d'un cathéter de perfusion selon l'invention ;
- les figures 10 et 11, des vues schématiques en plan de deux modes de réalisation d'une tubulure de perfusion selon l'invention ;
- la figure 12, une vue schématique en plan d'un autre mode de réalisation d'un dispositif de prélèvement selon l'invention, dans lequel la structure tubulaire inclut une partie du canal de prélèvement ; et
- les figures 13 et 14, des vues schématiques de deux modes de réalisation d'un réservoir de prélèvement selon l'invention.

L'exemple décrit par la suite se rapporte à une transfusion sanguine sécurisée. Cependant, l'invention couvre tout produit de perfusion devant être administré à un patient après un test de compatibilité avec le patient ou la maladie dont souffre le patient (urine, liquide céphalorachidien, liquide pleural, liquide d'ascite, liquide péritonéal, etc.).

Le système de perfusion sécurisé selon l'invention peut avantageusement être utilisé pour réaliser une transfusion sanguine sécurisée.

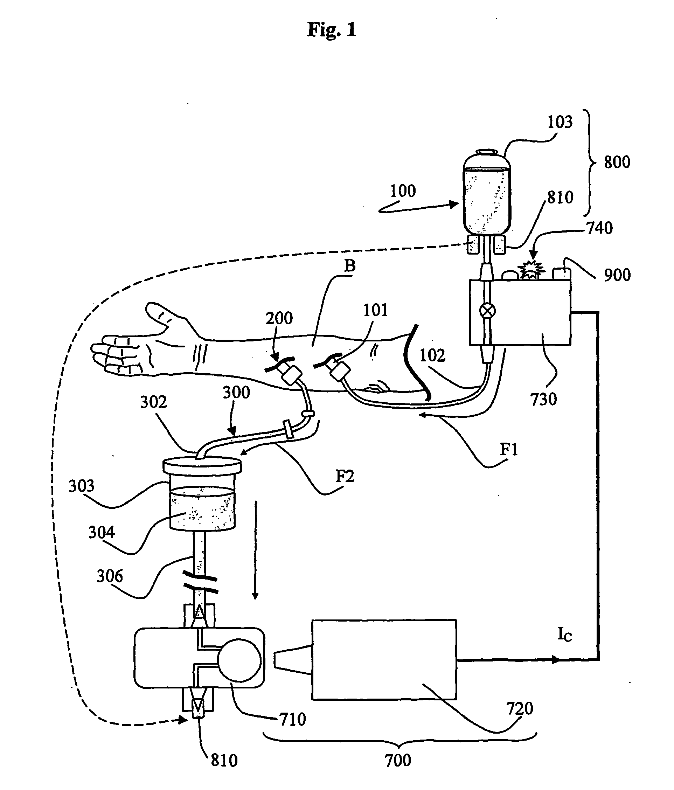

Ce système et sa mise en oeuvre son illustré aux figures 1 à 6a décrites ci-après.

Le système de perfusion sécurisé selon l'invention comprend, d'une manière générale, un circuit fluidique d'une première perfusion 100 comprenant un cathéter de perfusion 101, une tubulure de perfusion 102 et un réservoir 103 de produit déterminé de perfusion à administrer à un patient B. Un moyen de commande d'écoulement 730 du produit de perfusion dans la tubulure est associé à ce circuit pour autoriser ou interdire l'écoulement du produit.

Le positionnement du cathéter de perfusion dans le corps du patient ne fait pas partie de la présente invention.

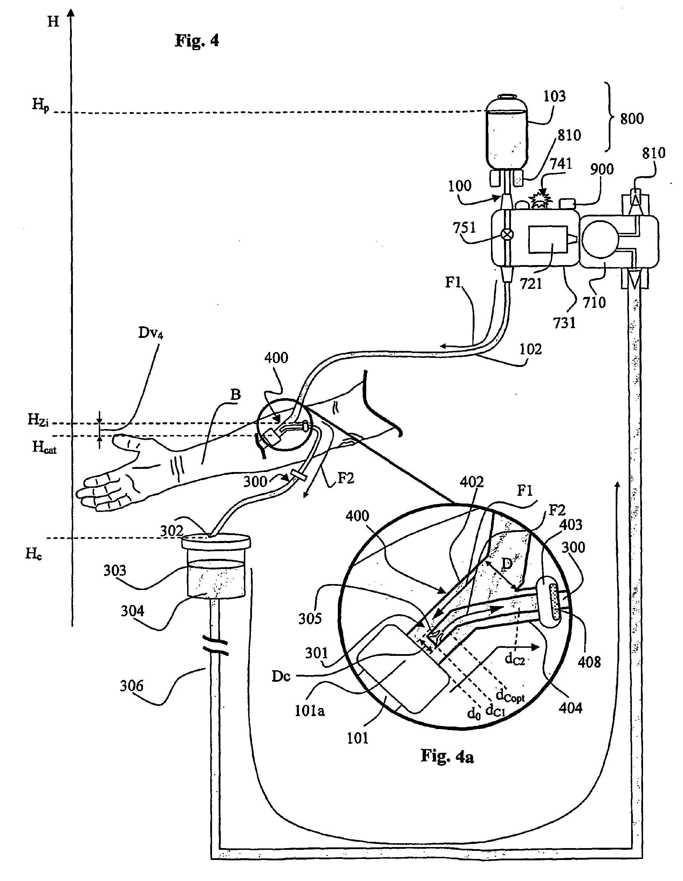

Le système de perfusion sécurisé selon l'invention comprend également un moyen de prélèvement 200 d'un liquide corporel d'un patient. Dans les figures 1 à 3, ce moyen de prélèvement comprend un cathéter classique 201, à installer au patient, de préférence dans son système vasculaire. Les figures 4 à 6a présentent un mode de réalisation particulier d'un moyen de prélèvement 200.

En utilisation, le moyen de prélèvement 200 est en liaison fluidique avec un réservoir de prélèvement 303 par l'intermédiaire d'un canal 300. Dans toute la suite de la présente description, les réservoirs de prélèvement présentent, avantageusement, une prise d'air obturable.

D'autre part, par l'intermédiaire d'une conduite 306, le réservoir de prélèvement 303 est en liaison fluidique avec un moyen d'analyse et de comparaison 700 du liquide corporel prélevé et d'un échantillon de produit déterminé. Dans l'exemple décrit d'une transfusion sanguine sécurisée selon l'invention, le produit déterminé est du sang stocké dans un des réservoirs secondaires 810 d'échantillonnage, séparables du réservoir principal 103 de la poche de transfusion 800.

Le moyen d'analyse 700 est apte à analyser le liquide corporel prélevé et l'échantillon de produit déterminé 810, ainsi qu'à les comparer entre eux. Le moyen d'analyse et de comparaison 700 est également apte à transmettre au moyen de commande d'écoulement 730 une information de comparaison I_{c} du liquide corporel prélevé 304 et de l'échantillon de produit déterminé 810.

La chambre de réaction 710 peut présenter toute forme adaptée à la réaction devant être détectée.

Un exemple de réalisation d'une chambre de réaction est une cassette comprenant une biopuce munie d'un circuit fluidique.

Un exemple particulièrement intéressant, dans le cadre d'un système de transfusion sanguine sécurisée, est l'utilisation de biopuces d'or biofonctionnalisées 710 en combinaison avec un dispositif de détection 720 à base de transductions optiques (tel que ceux commercialisés par la société GE Healthcare sous la dénomination « système BIAcore »).

Basé sur la résonance de plasmon de surface, ce type de dispositif mesure une variation d'angle d'extinction de résonance (dépendante d'une variation d'indice à l'interface or/diélectrique) qui peut être corrélée à une variation de masse.

Réalisée sur un support en verre muni d'une mince couche d'or, la fonctionnalisation se déroule en deux étapes. Une première étape permet la reconstitution d'un film mince organique présentant certaines fonctions chimiques activables (type SH, COOH, NH2....) pour le greffage ultérieur des anticorps. Lors de la seconde étape, cette couche est activée et les anticorps déposés à sa surface sont immobilisés.

Les inventeurs ont découvert qu'après traitement chimique de la surface d'or, l'immobilisation des anticorps anti-A et anti-B de type IgM était significativement améliorée à un pH d'environ 4,65.

Dans ces conditions de pH, le taux de greffage atteint en moyenne 1500 IgM/µm² ce qui permet d'impliquer jusqu'à 100 000 anticorps par globule rouge capturé.

Cela entraîne une forte interaction entre les hématies et l'immunocapteur présentant une surface fonctionnalisée par des IgM anti-A et anti-B, et ce même après plusieurs rinçages

Ainsi, le moyen 700 est capable d'analyser le sang prélevé 304 en interprétant les informations de détection, issues du moyen de détection 720, concernant les interactions entre les globules du sang et les anticorps présents sur la biopuce.

Cette interaction est très sensible et permet une analyse de compatibilité sanguine même en cas de pathologie ou d'antigènes faibles.

D'autres types d'interaction et d'autres moyens de détection que ceux précédemment décrit peuvent être utilisé pour analyser le sang (détection de virus, de protéines, de cellules rares circulantes, etc.) ou d'autres liquides corporels prélevés.

Dans le mode de réalisation de la figure 1, le moyen d'analyse 700 comprend une chambre de réaction 710 et un moyen de détection 720.

Le moyen de détection 720 du moyen d'analyse 700, localisé à proximité de la chambre de réaction 710, transmet une information de comparaison I_{c} à un moyen de commande d'écoulement 730 de la première perfusion. Le moyen de commande d'écoulement 730 est muni, de préférence, d'un moyen d'affichage 740 de l'information de comparaison.

De manière pratique, le moyen d'analyse 700 est, dans ce mode de réalisation, localisé en dessous du cathéter du patient, à proximité du réservoir de prélèvement 303.

Un tel mode de réalisation présente l'avantage de ne nécessiter qu'une faible longueur de conduites fluidiques (canal 300 et conduite 306). Cependant, l'encombrement global du système peut être important puisqu'il comprend un moyen d'analyse 700 et un moyen de commande d'écoulement 730 indépendant.

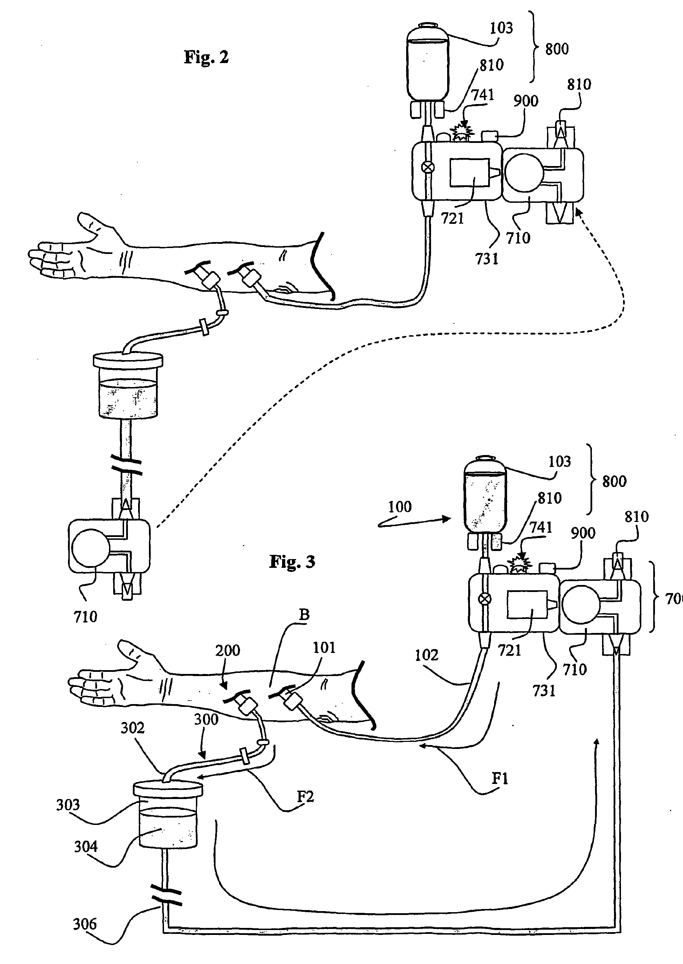

Dans le mode de réalisation illustré en figure 2, la chambre de réaction 710 est, comme dans la figure 1, connectée au réservoir 303 de liquide prélevé et à un échantillon 810 de produit déterminé.

Lorsque la chambre de réaction est prête pour l'analyse et la comparaison, l'opérateur la déconnecte de la conduite 306 (une vanne de fermeture, non représentée, est préférentiellement prévue à cet effet) et la connecte à un moyen de commande d'écoulement 731. Celui-ci est muni d'un moyen de détection et d'analyse 721 et d'un moyen d'affichage 741 de l'information de comparaison I_{c} (non représentée) émise par le moyen 721. La connexion de la chambre 710 sur le moyen de commande 731 peut être faite à l'aide d'un moyen de fixation (non représenté).

Le moyen de fixation peut être une pince prenant en sandwich la chambre de réaction 710, de telle sorte que la position de la chambre est optimale pour la détection et l'analyse par le moyen 721.

Ce mode de réalisation présente l'avantage d'être plus compact que celui illustré en figure 1. Cependant, il nécessite une étape de déconnexion fluidique de la chambre de réaction 710, ce qui peut constituer un risque de contact entre l'opérateur et le liquide prélevé.

Dans le mode de réalisation illustré en figure 3, le réservoir de prélèvement 303 est en communication fluidique, via une conduite 306, avec la chambre de réaction 710.

La conduite 306 est suffisamment longue pour permettre à un opérateur, lorsque la chambre de réaction est prête pour l'analyse et la comparaison, de placer la chambre 710 sur un moyen de fixation (non représenté) à un moyen de commande d'écoulement 731 semblable à celui décrit en figure 2. Celui-ci est muni d'un moyen de détection et d'analyse 721 et d'un moyen d'affichage 741 de l'information de comparaison I_{c} (non représentée) émise par le moyen 721.

Le moyen de fixation peut être une pince prenant en sandwich la chambre de réaction 710, de telle sorte que la position de la chambre est optimale pour la détection et l'analyse par le moyen 721.

Ce mode de réalisation évite de déconnecter la chambre de la conduite 306. Les risques de contact direct entre l'opérateur et le liquide prélevé sont donc diminués.

En outre, la détection et l'analyse se font sur la même ligne de perfusion. Puisqu'il y a une connexion entre la chambre 710 et le patient (via le dispositif de prélèvement 200-400, le canal 300, le réservoir 303 et la conduite 306), il devient impossible de faire une erreur de connexion entre la chambre 710 et le moyen de commande d'écoulement 731 ; il ne peut pas y avoir d'inversion de chambre de réaction 710 entre un patient et un autre patient.

Le système selon l'invention permet donc la réalisation d'un circuit fermé : le sang est prélevé, analysé et comparé à celui de la poche de transfusion et l'autorisation/interdiction d'écoulement est générée le long d'un même circuit bouclé sur le patient.

Dans les trois modes de réalisation précédents, lorsque l'analyse est opérée par le moyen d'analyse 700-720-721, celui-ci transmet au moyen de commande d'écoulement 730-731 de la deuxième perfusion, l'information de comparaison I_{c} du liquide corporel prélevé et de l'échantillon de produit déterminé.

Cette information est alors affichée grâce au moyen d'affichage 740-741.

Le personnel soignant sait, alors, si le produit de la première perfusion est compatible avec le patient et/ou la situation médicale qui a été diagnostiquée. Dans l'exemple de la transfusion sanguine, le personnel soignant sait si le sang de la poche de transfusion 800 est d'un groupe ABO compatible avec le groupe ABO du patient.

Dans un autre exemple, le personnel soignant pourrait déterminer si l'antibiotique présent dans la poche de perfusion ne provoque pas de réaction allergique avec le patient.

Lorsque l'information de comparaison I_{c} indique que le produit de la première transfusion est compatible avec le patient, le personnel soignant peut agir de plusieurs manières.

Selon un premier mode de réalisation, la poche de la première transfusion n'est pas encore en communication fluidique avec le patient. Dans ce cas, en fonction de l'information de comparaison I_{c}, le personnel soignant met, ou non, en communication fluidique la tubulure 102 et le réservoir 103 de la poche 800.

Ce mode de réalisation permet de ne connecter la poche 800 de produit de la première perfusion que si elle est compatible avec le patient. Cela évite de gâcher une poche de produit car ce système permet de récupérer la poche considérée comme incompatible.

Selon un deuxième mode de réalisation, la poche 800 est déjà en communication fluidique avec le reste du circuit de perfusion.

Pour que le produit ne s'écoule, le moyen de commande d'écoulement 730, 731 comprend une électrovanne 751.

Le moyen de commande d'écoulement 730, 731 est agencé pour bloquer l'écoulement du produit de la première perfusion si l'information de comparaison indique que le liquide corporel et le produit de la première perfusion ne sont pas compatibles, et autoriser l'écoulement du produit de la première perfusion si l'information de comparaison indique que le liquide corporel et le produit de la première perfusion sont compatibles.

Selon une première variante, cette électrovanne 751 est activable manuellement par un moyen manuel 900 de génération de l'écoulement.

Ainsi, en cas de compatibilité, le personnel soignant active le moyen manuel 900 de génération de l'écoulement dudit produit dans la tubulure 102. Puisque le produit de perfusion est compatible avec le patient, le moyen de commande d'écoulement 730, 731 autoriser l'écoulement du produit et ouvre l'électrovanne 751.

Si le personnel soignant prend, par mégarde, la décision de générer manuellement l'écoulement en activant le moyen de génération 900, alors que le produit de perfusion est incompatible avec le patient, le moyen de commande d'écoulement 730, 731 bloque l'écoulement du produit de la première perfusion en conservant l'électrovanne 751 fermée.

Selon une deuxième variante, l'électrovanne 751 est activable automatiquement par le moyen de commande d'écoulement 730, 731.

Il est également envisageable, pour certaines applications, que le moyen d'analyse soit aussi apte à contrôler le moyen de commande d'écoulement pour qu'il génère automatiquement l'écoulement du produit de la deuxième perfusion si le liquide corporel et le produit de la deuxième perfusion sont compatibles, et pour qu'il ne génère pas l'écoulement du produit de la deuxième perfusion si le liquide corporel et le produit de la deuxième perfusion ne sont pas compatibles.

Ainsi, en cas de compatibilité, le moyen de commande d'écoulement 730, 731 autoriser l'écoulement du produit et ouvre l'électrovanne 751.

Au contraire, en cas d'incompatibilité, le moyen de commande d'écoulement 730, 731 bloque l'écoulement du produit de la première perfusion en conservant l'électrovanne 751 fermée.

L'invention permet donc un contrôle ultime, sur la même ligne de perfusion connectée au patient. Il n'y a donc plus d'aléas entre l'analyse pour le contrôle ultime et la mise en oeuvre effective du traitement lui-même puisque le contrôle et l'autorisation de traitement sont liés directement au patient, sans intervention potentiellement erronée du personnel soignant.

Pour éviter de faire deux piqûres au patient, l'invention propose un système de perfusion sécurisé comprenant un moyen de prélèvement spécialement conçu pour permettre d'une part le prélèvement de liquide corporel et, d'autre part, l'écoulement du produit de la première perfusion 100. Ce mode de réalisation est illustré aux figures 4 et 4a.

Plus précisément, le moyen de prélèvement comprend un canal de prélèvement 300 comprenant une extrémité proximale 302 destinée à être reliée au réservoir de prélèvement 303, et une extrémité distale 301.

Le moyen de prélèvement comprend également un dispositif de prélèvement 400 destiné à être incorporé, non pas dans un patient, mais dans le circuit fluidique de la perfusion 100 préalablement installée sur un patient, entre le cathéter et le réservoir de perfusion, dans une partie externe du circuit fluidique par rapport au corps du patient.

Ce dispositif comprend une structure tubulaire 402 de connexion fluidique au circuit fluidique de la perfusion 100, munie d'une zone d'intubation, en utilisation, d'un canal de prélèvement 300 comprenant une extrémité distale 301.

Cette structure tubulaire comprend également un moyen de maintien 203. Celui-ci est adapté pour permettre, en utilisation, le maintien, dans la structure tubulaire, de la partie du canal de prélèvement 300 comprenant une extrémité distale 301, pour que l'extrémité distale 301 du canal de prélèvement 300 soit dirigée vers le cathéter de perfusion 101, dans le sens d'écoulement F1 du produit de perfusion 102, depuis un réservoir 101 de produit de perfusion vers le cathéter de perfusion 104.

Le terme « maintien » doit s'entendre au sens large. Il concerne, d'une part, un maintien réversible de la partie du canal de prélèvement insérée dans la structure tubulaire. Dans ce cas, le dispositif selon l'invention ne comprend pas de canal de prélèvement. Celui-ci doit être inséré par l'utilisateur, lors du prélèvement, dans la structure tubulaire (voir figures 4 à 11).

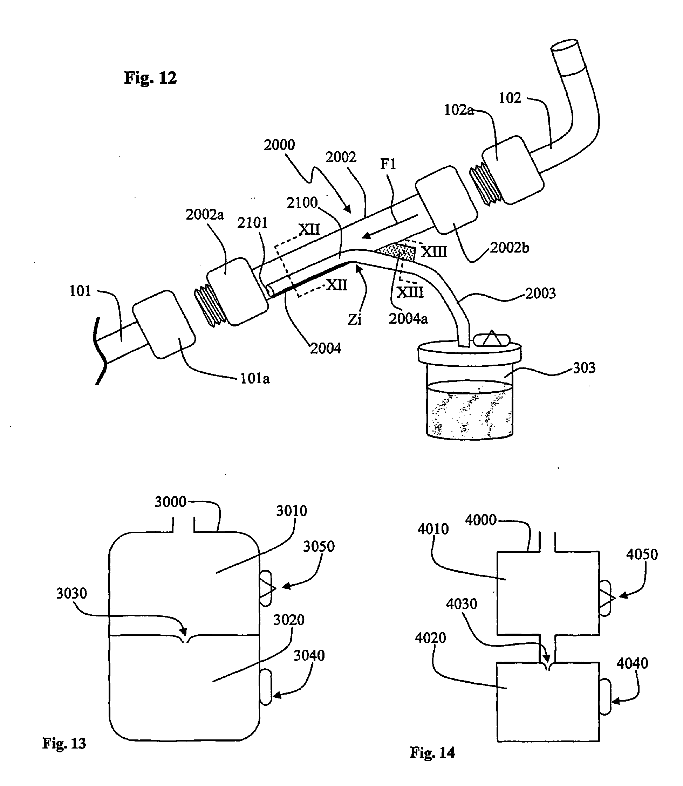

Le terme « maintien » se rapporte également à un maintien définitif dans la structure tubulaire, de la partie du canal de prélèvement pour qu'il ne forme qu'une seule et même pièce comprenant deux tubes. De cette manière, en utilisation, une partie du canal de prélèvement 1100 est agencée dans la structure tubulaire 1002 de sorte que l'extrémité distale 1101 du canal de prélèvement 1100 soit dirigée vers le cathéter de perfusion 101, dans le sens d'écoulement F1 du produit de perfusion, depuis un réservoir 103 de produit de perfusion vers le cathéter de perfusion 101 (voir figure 12).

Dans les modes de réalisation des figures 4 à 11, la zone d'intubation Zi comprend un moyen d'insertion.

Dans le mode de réalisation des figures 4 et 4a, la structure tubulaire 402 est un système de connexion en Y présentant une branche destinée à être en communication fluidique avec la tubulure 102 de la perfusion 100, et une branche 404 munie d'une zone d'intubation Zi portant un moyen de maintien 403 et une membrane 408 pour l'insertion étanche du canal 300. Cette membrane est en un matériau étanche conservant son étanchéité après avoir été percé.

Alternativement, la structure de la membrane (épaisseur et/ou rigidité) et son agencement dans le système en Y peuvent être tels que le canal de prélèvement est à la fois inséré et maintenu en position d'utilisation, c'est-à-dire à l'intérieur de la structure tubulaire 202. Dans ce cas, la membrane assure les fonctions d'un moyen de maintien et d'un moyen d'insertion. Autrement dit, le canal de prélèvement 300 est inséré et maintenu en position d'utilisation, c'est-à-dire à l'intérieur de la structure tubulaire 202, l'extrémité distale 301 du canal de prélèvement 300 étant dirigée vers le cathéter de perfusion 101, dans le sens d'écoulement F1 du produit de perfusion, depuis le réservoir 103 de produit de perfusion vers le cathéter de perfusion 101. Alternativement, la structure tubulaire peut comprendre un moyen de maintien indépendant de la membrane qui ne servirait, alors, qu'à l'insertion.

Le matériau étanche conservant son étanchéité après avoir été percé est choisi parmi un polymère de silicone, tel que le polydiméthylsiloxane (PDMS), du polyméthylmétacrylate (PMMA), du Polychlorure de vinyle (PVC), et du Tygon® (fabriqué par la société Saint-Gobain), etc.

Le canal de prélèvement 300 comprend une extrémité distale 301, qui présente une rigidité suffisante pour percer la membrane 408, et une extrémité proximale 302 destinée à être en communication fluidique avec un réservoir de prélèvement 303. Le réservoir de prélèvement de liquide présentent, avantageusement, une prise d'air obturable (non illustrée).

Selon une variante non illustrée, le moyen de maintien est un connecteur étanche, par exemple de type luer-lock. Dans ce cas, le canal 300 est équipé d'un connecteur luer-lock correspondant, dans lequel passe, de manière étanche, la partie du canal portant l'extrémité distale 301. L'association des connecteurs luer-lock permet l'insertion du canal de prélèvement dans la structure tubulaire et maintient la partie du canal portant l'extrémité distale 301 dirigée vers le cathéter de perfusion 101, dans le sens d'écoulement F1 du produit de perfusion, depuis un réservoir 103 de produit de perfusion vers le cathéter de perfusion 101.

En utilisation, la structure tubulaire 402 est connectée au cathéter 101 et à la tubulure 102. Cette connexion est préférentiellement réalisée au moyen de connecteurs luer-lock. Cette incorporation se fait à proximité de l'extrémité proximale 101a du cathéter de perfusion 101 et ne se fait donc pas directement dans le patient, mais dans une partie externe du circuit fluidique par rapport au corps du patient.

Dans un deuxième temps, on insère, via le moyen d'insertion 404 du dispositif de prélèvement 400, l'extrémité distale 301 du canal de prélèvement 300 dans la structure tubulaire 402 du dispositif de prélèvement, de telle sorte que ladite extrémité distale 301 soit dirigée vers le cathéter de perfusion 101, dans le sens d'écoulement F1 du produit de perfusion. L'extrémité distale 301 du canal de prélèvement 300 reste localisée à l'extérieure du cathéter de perfusion 101 et, donc, à l'extérieur du patient.

Un autre mode de réalisation d'un dispositif de prélèvement 2000 selon l'invention est illustré en figure 12. Il comprend une structure tubulaire 2002 de connexion au circuit fluidique de la perfusion représenté, dans cette figure 12, par un cathéter 101 et une tubulure 102. A cette fin, la structure tubulaire 2002, le cathéter 101 et la tubulure 102 présentent, de préférence, des connexions luer-lock, respectivement 2002a, 2002b, 101 a et 102a.

Le dispositif de prélèvement 2000 comprend également un moyen de maintien 2004 adapté pour permettre, en utilisation, le maintien, à l'intérieur de la structure tubulaire 2002, d'une partie d'un canal de prélèvement 2100 comprenant une extrémité distale 2101. De cette manière, en utilisation, l'extrémité distale 2101 du canal de prélèvement 2100 est dirigée vers le cathéter de perfusion 101, dans le sens d'écoulement F1 du produit de perfusion.

La partie du canal de prélèvement 2100 comprenant l'extrémité distale 2101 est donc directement incorporée dans la structure tubulaire 2002, et une partie 2003 du canal de prélèvement 2100 comprenant une extrémité proximale débouche hors la structure tubulaire au niveau de la zone d'intubation Zi.

Le moyen de maintien 2004 est ici constitué par la liaison mécanique entre le canal 2100 et la structure tubulaire. Le canal 2100 et la structure tubulaires 2002 constituent une seule et même pièce. Le moyen de maintien peut être une soudure longitudinale.

L'extrémité distale du canal de prélèvement est agencée à une distance d'abouchement déterminée de l'extrémité proximale du cathéter de perfusion 101, comprise entre 0 cm et 20 cm, de préférence entre 0 cm et 3 cm. Cette distance n'est pas réglable par le personnel soignant, mais prédéfinie lors de la fabrication du dispositif. Ceci permet une manipulation facilité en évitant un geste technique supplémentaire au personnel soignant.

Dans la figure 12, le moyen de maintien comprend, de manière optionnelle, une partie massive 2004a ressemblant à la branche en Y des modes de réalisation des figures 1 et 2. Cette partie massive permet de soutenir partiellement la partie 2003 débouchant de la partie tubulaire 2002 et, ainsi, éviter la plicature du canal 2100. Cette partie massive peut se situer au dessus, au dessous ou tout autour du canal de prélèvement.

Un dispositif de prélèvement selon l'invention incorporant directement une partie du canal de prélèvement évite à l'utilisateur d'avoir à insérer le canal dans la structure tubulaire. En effet, cette insertion est un geste médical qui peut ne pas être fait de manière satisfaisante, par exemple si la distance d'abouchement est trop importante. En outre, l'insertion peut engendrer une plicature du canal qui empêche l'écoulement du liquide corporel vers le réservoir 303.

Pour éviter ce phénomène de plicature, le canal de prélèvement 2100 peut comprendre au moins une structure en accordéon lui permettent de se courber sans formation de plis.

Cette structure en accordéon peut équiper le canal de prélèvement de tous les modes de réalisation du système de perfusion selon l'invention.

L'extrémité distale 301 du canal de prélèvement 300 reste localisée à l'extérieure du cathéter de perfusion et, donc, à l'extérieur du corps du patient.

Il s'agit donc d'une installation *ex vivo* du canal de prélèvement dans un circuit fluidique d'une perfusion située à l'extérieure du corps du patient. Il ne s'agit pas d'une installation *in vivo,* c'est-à-dire dans le corps du patient.

Ensuite, on clampe la tubulure de perfusion 102, puis on déclampe le canal de prélèvement 300 et on amène le réservoir de prélèvement 303 à une hauteur H_{c} inférieure à la hauteur H_{cat} du cathéter du patient. Avantageusement, on met le canal de prélèvement et le réservoir à la pression atmosphérique grâce à la prise d'air obturable du réservoir.

De manière surprenante, ceci permet au sang de s'écouler dans le canal de prélèvement 300, à contre-courant selon le sens de la flèche F2, par rapport au produit de perfusion qui est situé entre le cathéter 101 et le clamp de la tubulure 102. Le produit de perfusion situé au dessus du canal de prélèvement et en dessous du clamp de la tubulure 102 n'est pas prélevé en même temps que le sang.

En outre, on s'est aperçu qu'il n'est pas nécessaire d'abaisser le réservoir de perfusion 103 pour que le sang s'écoule dans le canal de prélèvement.

Lorsque le sang s'écoule dans le canal de prélèvement 300, on maintient le réservoir de prélèvement 303 dans cette position pendant une durée suffisante pour obtenir, dans le réservoir de prélèvement 303, le volume de prélèvement 304 souhaité.

Pour obtenir un écoulement du sang dans de bonnes conditions (vitesse d'écoulement, de confort du patient, et.), la zone d'intubation Zi du dispositif de prélèvement est agencée pour que, en position utilisation, elle soit disposée à une distance verticale maximale déterminée Dvₘₐₓ de l'extrémité proximale du cathéter 101. La direction verticale est la direction du champ de pesanteur.

La distance verticale Dv est définie comme la distance entre la hauteur H_{zi} de la zone d'intubation et la hauteur H_{cat} du cathéter de perfusion sur l'échelle de hauteur H.

La distance verticale maximale Dvₘₐₓ est inférieure à 50 cm. De préférence, elle est comprise entre -50 et 50 cm (le signe négatif signifie que la zone d'intubation est en dessous de l'extrémité proximale du cathéter).

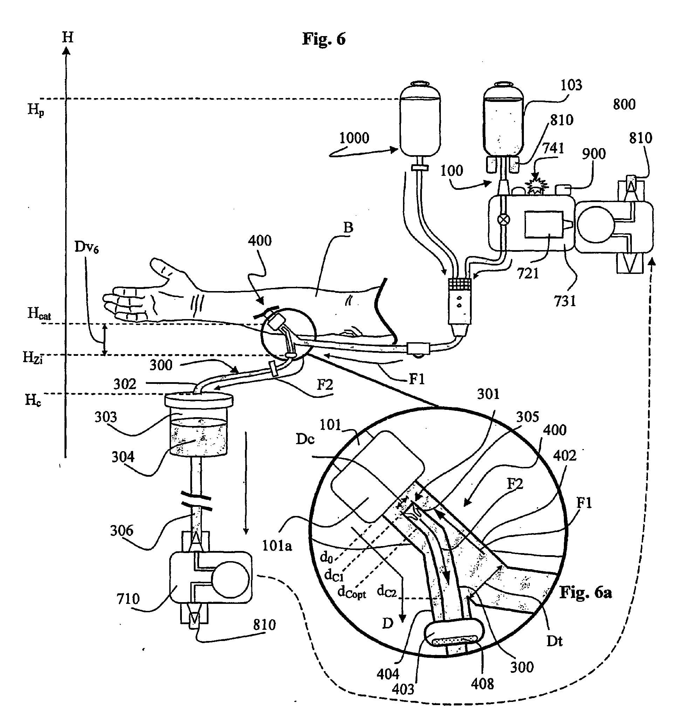

Sur les figures 6 à 7a, la zone d'intubation Zi est située sous le cathéter de perfusion 101. La distance verticale, notée respectivement Dv6 et Dv7, est donc négative. Cette disposition est optimale pour obtenir l'écoulement sanguin.

Cependant, dans la pratique, il peut arriver que le cathéter de perfusion 101 soit situé sous la zone d'intubation Zi. Cette situation, illustrée aux figures 4 à 5a, peut arriver, par exemple lorsque le dispositif de perfusion selon l'invention est disposé dans la boucle de sécurité. Dans ce cas, la distance verticale Dv4-Dv5 est positive.

Pour obtenir un écoulement du sang, la distance Dv4-Dv5 doit rester inférieure à la distance verticale maximale déterminée Dvₘₐₓ.

Dans la pratique, et de manière générale, le dispositif de prélèvement est incorporé dans un circuit fluidique de la perfusion à proximité du cathéter de perfusion, c'est-à-dire de telle sorte que la zone d'intubation soit disposée à une distance comprise entre 0 et 50 cm, de préférence entre 5 cm et 15 cm.

De cette manière, lors de l'utilisation, la zone d'intubation a peu de risque de se trouver au dessus de l'extrémité proximale du cathéter, à un distance verticale supérieure à la distance verticale maximale déterminée Dvₘₐₓ.

Au tout début du prélèvement (régime transitoire), le liquide prélevé est constitué par un mélange de sang et du produit de perfusion situé entre le système vasculaire du patient et l'extrémité distale 301 du canal de prélèvement 300.

Il peut donc être préférable de ne connecter le réservoir de prélèvement 303 que lorsque le sang paraît pur (visuellement ou par tout moyen d'analyse).

Alternativement, on peut connaitre le volume de produit de perfusion entre le système vasculaire du patient et l'extrémité distale 301 du canal de prélèvement 300 grâce aux dimensions du cathéter et à la mesure de la distance d_{c1} dite « distance d'abouchement » entre l'extrémité proximale 101a du cathéter 101 (déterminant l'origine d₀ du repère de distance D sur les figures 1 et 2) et l'extrémité distale 301 du canal 300. On peut donc calculer le taux de dilution du sang prélevé dans le réservoir 303.

Selon un autre mode de réalisation, on peut prévoir un réservoir 3000 à deux compartiments 3010 et 3020 séparés par un système anti-retour, tel qu'une valve 3030, un clapet, une bille, un flotteur etc (voir figure 13). Selon une variante, les deux compartiments 4010 et 4020 du réservoir 4000 sont montés en série de manière indépendante et séparés par un système anti retour 4030 (voir figure 14). Dans les deux exemples illustrés, le compartiment 3020 ou le compartiment 4020 le plus en aval doit permettre le stockage d'une quantité de liquide corporel égale, ou légèrement supérieure, au volume de produit de perfusion situé entre le système vasculaire du patient et l'extrémité distale 301 du canal de prélèvement 300. Au cours de ce stockage, l'air est évacué par une valve d'évacuation d'air 3040 - 4040.

Lorsque le compartiment 3020 ou le compartiment 4020 le plus en aval est rempli, le compartiment 3010 ou le compartiment 4010 amont se remplit. Les systèmes anti retour 3030-4030 évitent que ce liquide dilué, situé dans le compartiment le plus en aval, ne se mélange au liquide « pur » qui est stocké dans le compartiment amont.

Une analyse ultérieure pourra donc être préférentiellement menée sur le liquide pur. A cette fin, les réservoirs des figures 13 et 14 présentent une valve 3050-4050 d'évacuation d'air et de connexion à un moyen d'analyse du liquide corporel prélevé (non représenté sur ces figures).

Une fois le liquide corporel prélevé et stocké dans le réservoir 303, il est analysé comme décrit précédemment.

Si le liquide corporel et le produit de perfusion sont compatibles, le moyen de commande d'écoulement 731 autorise l'écoulement du produit de perfusion dans la tubulure 102 puis dans le patient via le cathéter 101.

Il n'est donc pas nécessaire de réaliser deux piqûres au patient pour le prélèvement et la perfusion.

Lorsqu'un patient est déjà perfusé, par exemple avec un liquide thérapeutique, il peut être nécessaire de lui faire une deuxième perfusion d'un autre liquide, tel que du sang, après que le médecin ait diagnostiqué une situation médicale donnée (maladie, accident, hémorragie, etc.).

Selon un mode de réalisation avantageux de l'invention, le système de perfusion sécurisé peut être mis en oeuvre alors que le patient est déjà équipé d'une première perfusion 1000, sans qu'il soit nécessaire de réaliser une piqûre supplémentaire au patient ni qu'aucune intervention chirurgicale directe sur le patient pour la mise en place du dispositif selon l'invention, ou pour sa mise en oeuvre.

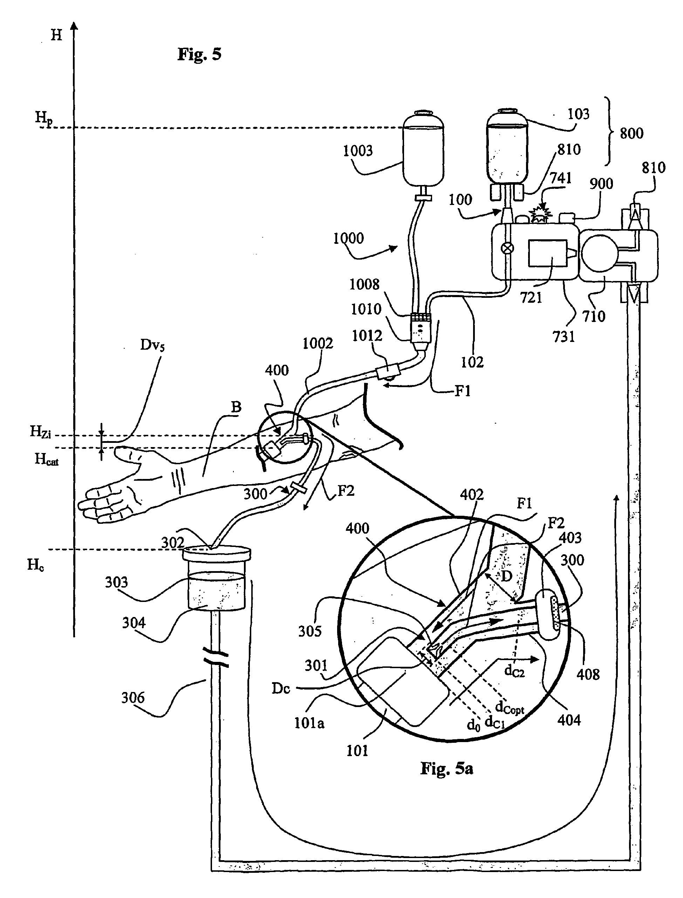

Ce mode de réalisation est illustré aux figures 5 à 7a. À cette fin, le système de perfusion sécurisé selon l'invention comprend un moyen de prélèvement spécialement conçu pour permettre d'une part l'écoulement des produits de la première perfusion 1000 et de la deuxième perfusion 100, mais également pour réaliser un prélèvement de liquide corporel.

La première perfusion 1000 comprend un circuit fluidique comprenant une tubulure de perfusion 1002 et un réservoir 1003 de produit déterminé de perfusion à administrer à un patient B.

Entre la tubulure 1002 et le réservoir 1003, sont préférentiellement prévus un filtre 1008, un vase d'expansion 1010 et une molette de réglage 1012 du débit du produit de perfusion.

La tubulure 1002 est reliée au système vasculaire du patient par le un cathéter de perfusion 101.

Pour connecter la deuxième perfusion 100 au système vasculaire du patient sans piqûre additionnelle, le filtre 1008 comprend un moyen de connexion d'une deuxième tubulure 102.

Alternativement, la tubulure 1002 de la première perfusion peut comprendre un moyen de connexion de la deuxième tubulure 102.

Un moyen de commande d'écoulement 731 du produit de perfusion est associé à la tubulure 102 pour autoriser ou interdire l'écoulement du produit de la deuxième perfusion 100.

Comme dans le mode de réalisation illustré aux figures 4 et 4a, le système de perfusion sécurisé selon l'invention, illustré aux figures 5 et 5a, comprend un dispositif de prélèvement 400 destiné à être incorporé dans le circuit fluidique de la première perfusion 1000. Ce dispositif comprend une structure tubulaire 402 de connexion fluidique à la tubulure 1002 de la première perfusion 1000, munie d'une d'intubation Zi portant un moyen de maintien 403 et une membrane 408 pour l'insertion étanche du canal 300. Le moyen de maintien 403 est adapté pour permettre, en utilisation, le maintien, dans la structure tubulaire 402, d'une partie du canal de prélèvement 300. La membrane est en un matériau étanche conservant son étanchéité après avoir été percé. Alternativement, la structure de la membrane (épaisseur et/ou rigidité) et son agencement dans le système en Y peuvent être tels que le canal de prélèvement est à la fois inséré et maintenu en position d'utilisation, c'est-à-dire à l'intérieur de la structure tubulaire 202. Dans ce cas, la membrane assure les fonctions d'un moyen de maintien et d'un moyen d'insertion.

Lorsqu'un prélèvement de liquide corporel est nécessaire, ici du sang, on incorpore le dispositif de prélèvement 400 selon l'invention dans le circuit fluidique de la première perfusion 1000 préalablement installée sur un patient B.

Cette incorporation se fait à proximité de l'extrémité proximale 101a du cathéter de perfusion 101 et ne se fait donc pas directement dans le patient, mais dans une partie externe du circuit fluidique par rapport au corps du patient.

Il n'est donc pas nécessaire de réaliser une piqûre supplémentaire au patient. Aucune intervention chirurgicale directe sur le patient n'est nécessaire pour la mise en place du dispositif selon l'invention, ni pour sa mise en oeuvre.

Ensuite, on insère, via le moyen d'insertion 404 du dispositif de prélèvement 400, l'extrémité distale 301 du canal de prélèvement 300 dans la structure tubulaire 402 du dispositif de prélèvement, de telle sorte que ladite extrémité distale 301 soit dirigée vers le cathéter de perfusion 101, dans le sens d'écoulement F1 du produit de perfusion. L'extrémité distale 301 du canal de prélèvement 300 reste localisée à l'extérieure du cathéter de perfusion 101 et, donc, à l'extérieur du patient.

Cette étape d'insertion est inutile si l'on utilise un dispositif de prélèvement incorporant déjà une partie du canal de prélèvement.

Ensuite, on amène le réservoir de prélèvement 303 à une hauteur H_{c} inférieure à la hauteur H_{cat} du cathéter du patient ce qui permet au sang de s'écouler dans le canal de prélèvement, à contre-courant selon le sens de la flèche F2. Le produit de perfusion situé au dessus du canal de prélèvement et en dessous du clamp de la tubulure 102 n'est pas prélevé en même temps que le sang.

Le prélèvement se passe de la même manière que pour l'installation illustrée en figures 4 et 4a.

Avantageusement, pour éviter, par exemple lors d'une mauvaise manipulation, un retour de sang depuis le canal de prélèvement vers le circuit fluidique de la perfusion, l'extrémité distale 301 du canal de prélèvement 300 est munie d'un moyen anti retour 305 tel qu'une valve anti retour de type fente de canard ou tricuspide. Alternativement, le moyen anti-retour peut également être situé à l'extrémité proximale du canal de prélèvement 300.

Ensuite, la mise en oeuvre du système de perfusion sécurisé est semblable à ceux décrit aux figures 1 à 3 et permet les mêmes avantages.

Ainsi, avec le mode de réalisation illustré aux figures 4 à 5a, le réservoir de prélèvement 303 est en communication fluidique, via une conduite 306, avec la chambre de réaction 710.

La conduite 306 est suffisamment longue pour permettre à un opérateur, lorsque la chambre de réaction est prête pour l'analyse et la comparaison, de placer la chambre 710 sur un moyen de fixation (non représenté) à un moyen de commande d'écoulement 731. Celui-ci est muni d'un moyen de détection et d'analyse 721 et d'un moyen d'affichage 741 de l'information de comparaison I_{c} (non représentée) émise par le moyen 721.

Le moyen de fixation peut être une pince prenant en sandwich la chambre de réaction 710, de telle sorte que la position de la chambre est optimale pour la détection et l'analyse par le moyen 721.

Ce mode de réalisation évite de déconnecter la chambre de la conduite 306. Les risques de contact direct entre l'opérateur et le liquide prélevé sont donc diminués.

De même, avec le mode de réalisation illustré aux figures 6 et 6a, la chambre de réaction 710 est, comme dans la figure 2, connectée au réservoir 303 de liquide prélevé et à un échantillon 810 de produit déterminé.

Lorsque la chambre de réaction est prête pour l'analyse et la comparaison, l'opérateur la déconnecte de la conduite 306 (une vanne de fermeture, non représentée, est préférentiellement prévue à cet effet) et la connecte à un moyen de commande d'écoulement 731. Celui-ci est muni d'un moyen de détection et d'analyse 721 et d'un moyen d'affichage 741 de l'information de comparaison I_{c} (non représentée) émise par le moyen 721. La connexion de la chambre 710 sur le moyen de commande 731 peut être faite à l'aide d'un moyen de fixation (non représenté).

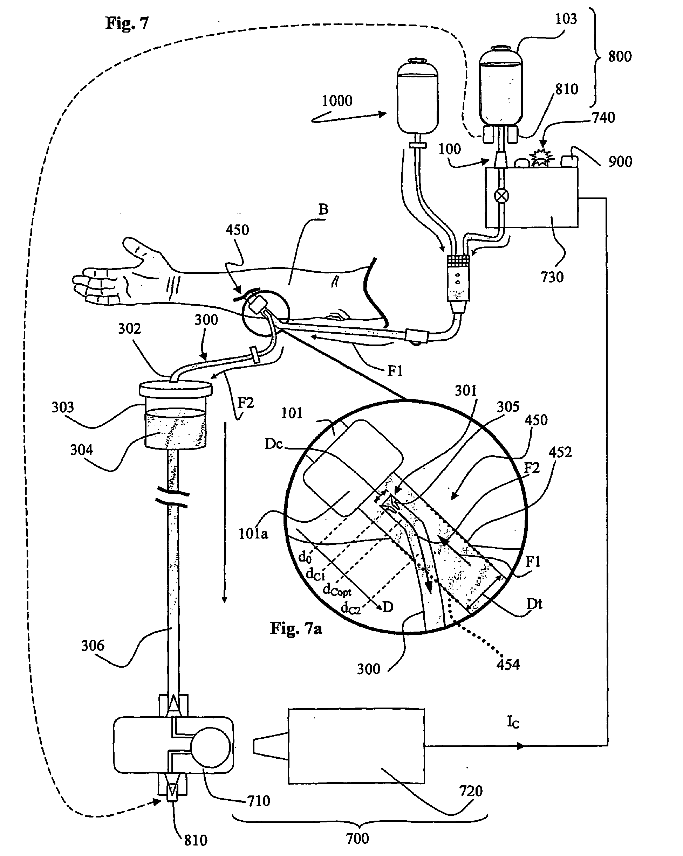

Enfin, avec le mode de réalisation illustré aux figures 7 et 7a, le moyen de détection 720 du moyen d'analyse 700, localisé à proximité de la chambre de réaction 710, transmet une information de comparaison I_{c} à un moyen de commande d'écoulement 730 de la première perfusion. Le moyen de commande d'écoulement 730 est muni, de préférence, d'un moyen d'affichage 740 de l'information de comparaison.

De manière pratique, le moyen d'analyse 700 est, dans ce mode de réalisation, localisé en dessous du cathéter du patient, à proximité du réservoir de prélèvement 303.

Un tel mode de réalisation présente l'avantage de ne nécessiter qu'une faible longueur de conduites fluidiques (canal 300 et conduite 306). Cependant, l'encombrement global du système peut être important puisqu'il comprend un moyen d'analyse 700 et un moyen de commande d'écoulement 730 indépendant.

Les figures 7 et 7a illustrent également un autre mode de réalisation d'un dispositif de prélèvement 450 selon l'invention.

Sur ces figures, le dispositif de prélèvement de liquide 450 selon l'invention comprend une structure tubulaire 452 de connexion au circuit fluidique de la perfusion 1000 munie d'une zone d'intubation Zi comprenant un moyen d'insertion 454, dans la structure tubulaire 452, d'une partie d'un canal de prélèvement 300.

Dans ce mode de réalisation, le moyen d'insertion 454 est une membrane en un matériau étanche conservant son étanchéité après avoir été percé. Cette membrane 454 peut occuper toute ou partie de la paroi de la structure tubulaire 452. Cette membrane 454 présente une structure (épaisseur et/ou rigidité et/ou matériau et/ou agencement) qui lui permet d'assurer à la fois la fonction de moyen de maintien et la fonction de moyen d'insertion. Autrement dit, le canal de prélèvement 300 est inséré et maintenu en position d'utilisation, c'est-à-dire à l'intérieur de la structure tubulaire 452, l'extrémité distale 301 du canal de prélèvement 300 étant dirigée vers le cathéter de perfusion 101, dans le sens d'écoulement F1 du produit de perfusion, depuis le réservoir 1003 de produit de perfusion vers le cathéter de perfusion 101. Alternativement, la structure tubulaire peut comprendre un moyen de maintien indépendant de la membrane qui ne servirait, alors, qu'à l'insertion.

Le dispositif de prélèvement selon l'invention peut être incorporé dans le circuit fluidique de la perfusion entre le cathéter de perfusion et la tubulure de perfusion. Pour cela, le dispositif de prélèvement comprend :
- un moyen de connexion à une extrémité proximale du cathéter de perfusion, et
- un moyen de connexion à l'extrémité distale de la tubulure de perfusion.

Pour installer ce dispositif, l'opérateur débranche l'extrémité proximale du cathéter de perfusion et l'extrémité distale de la tubulure de perfusion, et connecte le dispositif.

Les moyens de connexion peuvent être du type luer-lock.

Ce mode de réalisation implique, néanmoins, de débranche temporairement la perfusion:

Il peut être préférable de prévoir un cathéter ou une tubulure de perfusion pré-équipés, lors de leur fabrication, d'un dispositif de prélèvement selon l'invention.

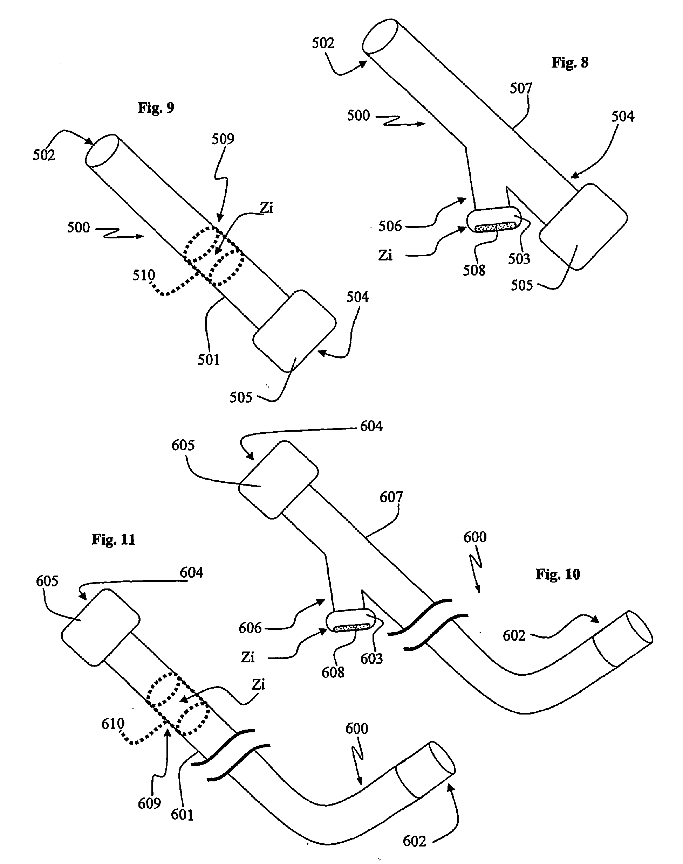

Ainsi, comme le montrent les figures 8 et 9, un cathéter de perfusion 500 selon l'invention comprend :
- une extrémité distale 502 destinée à être insérée dans un patient ;
- une extrémité proximale 504 destinée à être connectée, par exemple par un connecteur luer-lock 505, avec une extrémité distale d'une tubulure de perfusion ; et
- entre l'extrémité distale 502 et l'extrémité proximale 504, un dispositif 506-509 de prélèvement de liquide corporel selon l'invention, agencé pour que, en utilisation, la zone d'intubation Zi soit disposée à une distance verticale maximale déterminée Dvₘₐₓ. de l'extrémité proximale du cathéter.

Cet agencement permet un bon écoulement du liquide corporel lors de l'utilisation. La distance verticale maximale déterminée Dvₘₐₓ est comprise entre 0 cm et 50 cm.

Dans ces figures, l'aiguille de perforation du cathéter n'a pas été représentée.

Dans le mode de réalisation de la figure 8, le dispositif 506 présente une structure tubulaire 507 qui est un système de connexion en Y comprenant un moyen de maintien 503 muni d'une membrane d'insertion 508 en un matériau étanche conservant son étanchéité après avoir été percé. Cette structure tubulaire est semblable à celle qui a été décrite en relation avec les figures 4 à 6a.

Dans le mode de réalisation de la figure 9, le dispositif 509 présente une structure tubulaire 501 muni d'une membrane 510 en un matériau étanche conservant son étanchéité après avoir été percé. Cette membrane 510 présente une structure (épaisseur et/ou rigidité et/ou matériau et/ou agencement) qui lui permet d'assurer à la fois le rôle de moyen de maintien et le rôle de moyen d'insertion. Cette membrane 510 est semblable à celle qui a été décrite en relation avec les figures 7 et 7a.

Ainsi, lorsque la perfusion est mise en place avec ce cathéter selon l'invention, le dispositif de prélèvement selon l'invention est incorporé dans le circuit fluidique de la perfusion entre le cathéter de perfusion et la tubulure de perfusion.

De même, comme le montrent les figures 10 et 11, une tubulure de perfusion 600 selon l'invention comprend :
- une extrémité proximale 602 destinée à être connectée à un réservoir d'un premier produit de perfusion ;
- une extrémité distale 604 destinée à être connectée, par exemple par un connecteur luer-lock 605, avec une extrémité proximale d'un cathéter de perfusion insérée dans un patient ; et
- entre l'extrémité proximale 602 et l'extrémité distale 604, un dispositif 606-609 de prélèvement de liquide corporel selon l'invention, agencé pour que, en utilisation, la zone d'intubation soit disposée à une distance verticale maximale déterminée Dvₘₐₓ de l'extrémité proximale du cathéter de perfusion.

Ce dispositif de prélèvement est, de préférence, agencé au plus proche de l'extrémité distale 604, pour que, en position d'utilisation, la zone d'intubation soit disposée à une distance comprise entre 0 cm et 50 cm, de préférence entre 5 et 15 cm.

Dans le mode de réalisation de la figure 10, le dispositif 606 présente une structure tubulaire 607 qui est un système de connexion en Y comprenant un moyen de maintien 603 muni d'une membrane d'insertion 608 en un matériau étanche conservant son étanchéité après avoir été percé. Cette structure tubulaire est semblable à celle qui a été décrite en relation avec les figures 4 à 6a.

Dans le mode de réalisation de la figure 11, le dispositif 609 présente une structure tubulaire 601 muni d'une membrane 610 en un matériau étanche conservant son étanchéité après avoir été percé. Cette membrane 610 présente une structure (épaisseur et/ou rigidité et/ou matériau et/ou agencement) qui lui permet d'assurer à la fois le rôle de moyen de maintien et le rôle de moyen d'insertion. Cette membrane 610 est semblable à celle qui a été décrite en relation avec les figures 7 et 7a.

Ainsi, lorsque la perfusion est mise en place, avec cette tubulure selon l'invention, le dispositif de prélèvement selon l'invention est incorporé dans le circuit fluidique de la perfusion entre le cathéter de perfusion et la tubulure de perfusion.

Avec un cathéter ou une tubulure intégrant, dès la fabrication, un dispositif de prélèvement selon l'invention, il n'est pas nécessaire d'arrêter temporairement la perfusion pour incorporer le dispositif de prélèvement selon l'invention.

Dans tous les systèmes de perfusion sécurisés exposés aux figures 4 à 11, le rapport entre le diamètre Dc du canal de prélèvement et le diamètre Dt de la structure tubulaire du dispositif de prélèvement est, de préférence, inférieur à 1, et typiquement compris entre 1/20 et 1/3. Ceci permet d'assurer la continuité de l'apport en produit de perfusion malgré le prélèvement sanguin.

De même, le diamètre externe du cathéter de perfusion est compris, de préférence, entre 14 G (gauge) et 24 G, avantageusement entre 14 G et 18 G.

Plus ce diamètre est important, plus le débit de prélèvement peut être important. En jouant sur la hauteur du réservoir par rapport au cathéter du patient, on peut régler la vitesse d'écoulement et augmenter le débit. Le prélèvement peut donc se faire en un temps confortable pour le patient, de dix secondes ou moins.

De préférence, le canal de prélèvement présente une longueur entre ses deux extrémités comprise entre 10 cm et 100 cm, avantageusement entre 20 cm et 50 cm.

Selon un autre mode de réalisation non illustré, le dispositif de prélèvement comprend un réservoir, avantageusement muni d'une prise d'air obturable, relié à une tubulure connectable à son extrémité distale par une liaison de type luer-lock. Ce système peut être mis en place sur un robinet trois voies déjà en place, disposé sur un prolongateur ou sur une rampe de robinet connecté d'une part au cathéter en place sur le patient et d'autre part à la tubulure de perfusion. Le prélèvement est alors réalisé en abaissant le réservoir, comme décrit précédemment. Ce mode de réalisation présente l'avantage d'être très simple. Néanmoins, il entraine un volume dilué (sang et liquide de perfusion) et un temps de remplissage plus importants qu'avec les modes de réalisation décrits précédemment.

Le procédé selon l'invention n'a pas pour objet de diagnostiquer la situation médicale (maladie, accident, hémorragie, etc.) et/ou de choisir le traitement ad hoc puisque ces étapes ont préalablement été effectuées par le médecin, en fonction de la situation médicale et du patient.

Le procédé vise à s'assurer que le traitement qui va être prodigué est bien conforme au choix du médecin. Par exemple, le médecin a diagnostiqué la situation médicale X et il a choisi de traiter le patient Y avec le produit Z qui est compatible avec la situation médicale X et le patient Y. Après ce diagnostique, le procédé selon l'invention permet d'effectuer un contrôle ultime lors de la mise en oeuvre du traitement, en vérifiant que c'est bien le produit Z, compatible avec la situation médicale X et le patient Y, qui est appliqué.

Pour l'exemple du sang, le procédé selon l'invention permet d'effectuer un contrôle ultime lors de la mise en oeuvre de la transfusion, en vérifiant que le sang de la poche de transfusion, nécessaire au traitement de la situation médicale X préalablement diagnostiquée, est compatible avec le groupe ABO du sang du patient Y.

Ce procédé ne vise donc pas à diagnostiquer une situation médicale nécessitant une transfusion sanguine ou à éviter une situation médicale nécessitant une transfusion sanguine.

C'est une méthode visant à éviter un accident médical (incompatibilité entre le produit de traitement et le patient) et non visant à traiter un état pathologique.

## Revendications

1. Système de perfusion sécurisé d'un liquide corporel, **caractérisé en ce qu'**il comprend :
- un circuit fluidique d'une perfusion (100, 1000) comprenant un cathéter de perfusion (101), une tubulure de perfusion (102, 1002) et un réservoir (103, 1003) de produit de perfusion déterminé à perfuser à un patient (B) ;
- un moyen de prélèvement (200, 400) d'un liquide corporel d'un patient,
- un réservoir de prélèvement (303, 3000, 4000) muni d'un moyen de liaison fluidique (300) avec le moyen de prélèvement (200, 400),
- un moyen d'analyse et de comparaison (700, 710, 720, 721) du liquide corporel prélevé et d'un échantillon (810) de produit déterminé,
- un moyen de liaison fluidique entre le moyen d'analyse et le réservoir de prélèvement ;
- un moyen de commande d'écoulement (730, 731) du produit de perfusion dans la tubulure, le moyen d'analyse et de comparaison (700, 720) étant apte à transmettre au moyen de commande d'écoulement (730, 731) une information de comparaison (I_{c}) du liquide corporel prélevé (304) et de l'échantillon de produit déterminé (810).

2. Système de perfusion sécurisé selon la revendication 1, dans lequel le moyen d'analyse (700, 720) est apte à contrôler le moyen de commande d'écoulement (730, 731) pour qu'il bloque l'écoulement du produit de perfusion si l'information de comparaison (I_{c}) indique que le liquide corporel et le produit de perfusion ne sont pas compatibles, et pour qu'il autorise l'écoulement du produit de perfusion si l'information de comparaison (I_{c}) indique que le liquide corporel et le produit de perfusion sont compatibles.

3. Système de perfusion sécurisé selon l'une quelconque des revendications 1 ou 2, comprenant un moyen d'affichage (740, 741) de l'information de comparaison (I_{c}) du liquide corporel prélevé (304) et de l'échantillon de produit déterminé (810).

4. Système de perfusion sécurisé selon l'une quelconque des revendications 1 à 3, dans lequel le moyen d'analyse (700, 720) est apte à contrôler le moyen de commande d'écoulement (730, 731) pour qu'il génère automatiquement l'écoulement du produit de perfusion si le liquide corporel et le produit de perfusion sont compatibles, et pour qu'il ne génère pas l'écoulement du produit de perfusion si le liquide corporel et le produit de perfusion ne sont pas compatibles.

5. Système de perfusion sécurisé selon l'une quelconque des revendications 1 à 4, dans lequel le moyen d'analyse (700) comprend une chambre de réaction (710) et un moyen de détection (720, 721).

6. Système de perfusion sécurisé selon l'une quelconque des revendications 1 à 5, dans lequel le moyen de prélèvement comprend :
- un canal de prélèvement (300, 2100) comprenant une extrémité proximale (302, 2003) destinée à être reliée au réservoir de prélèvement (303), et une extrémité distale (301, 2101), et
- un dispositif de prélèvement (400, 450, 500, 600, 2000) destiné à être incorporé dans le circuit fluidique d'une perfusion (100-1000), et comprenant une structure tubulaire (402, 452, 507, 607, 2002) de connexion au circuit fluidique de la perfusion, munie :
∘ d'une zone d'intubation (Zi), en utilisation, d'un canal de prélèvement (300, 2100) comprenant une extrémité distale (301,2101) ; et
∘ d'un moyen de maintien (403, 408, 454, 503, 510, 603, 610, 2004, 2004a), en utilisation, dans la structure tubulaire, de la partie du canal de prélèvement (300, 2100) comprenant une extrémité distale (301, 2101), de sorte que, en utilisation, ladite extrémité distale (301, 2101) soit dirigée vers le cathéter de perfusion (101), dans le sens d'écoulement (F1) du produit de perfusion (102), depuis un réservoir (103) de produit de perfusion vers le cathéter de perfusion (101).

7. Système de perfusion sécurisé selon la revendication 6, dans lequel la zone d'intubation est agencée pour que, en utilisation, elle soit disposée à une distance verticale maximale déterminée (Dvₘₐₓ) de l'extrémité proximale du cathéter.

8. Système de perfusion sécurisé selon la revendication 7, dans lequel la distance verticale maximale déterminée (Dvₘₐₓ) est comprise entre 0 cm et 50 cm.

9. Système de perfusion sécurisé selon l'une quelconque des revendications 6 à 8, dans lequel la partie comprenant l'extrémité distale (1101) du canal de prélèvement (1100) est fixée dans la structure tubulaire (1002), et une partie (1003) comprenant une extrémité proximale du canal de prélèvement (1100) débouche hors la structure tubulaire (1002) au niveau de la zone d'intubation (Zi).

10. Système de perfusion sécurisé selon l'une quelconque des revendications 6 à 8, dans lequel la zone d'intubation comprend un moyen d'insertion (408, 454, 508, 510, 608, 610) adapté pour permettre, en utilisation, l'insertion, dans la structure tubulaire, de la partie du canal de prélèvement (300) comprenant l'extrémité distale (301).

11. Système de perfusion sécurisé selon la revendication 10, dans lequel le moyen d'insertion est choisi parmi :
- une membrane (408, 454, 508, 510, 608, 610) en un matériau étanche conservant son étanchéité après avoir été percé ; et
- un connecteur étanche.

12. Système de perfusion sécurisé selon la revendication 11, dans lequel le matériau étanche conservant son étanchéité après avoir été percé est choisi parmi un polymère de silicone, tel que le polydiméthylsiloxane (PDMS), du polyméthylmétacrylate (PMMA), et du Polychlorure de vinyle (PVC).

13. Système de perfusion sécurisé selon l'une quelconque des revendications 6 à 12, dans lequel le dispositif de prélèvement comprend, en outre :
- un moyen de connexion à une extrémité proximale du cathéter de la perfusion, et
- un moyen de connexion à une extrémité distale de la tubulure de la perfusion.

14. Système de perfusion sécurisé selon l'une quelconque des revendications 6 à 13, dans lequel le canal de prélèvement (300) présente une longueur entre ses deux extrémités comprise entre 10 cm et 100 cm, de préférence entre 20 cm et 50 cm.

15. Système de perfusion sécurisé selon l'une quelconque des revendications 6 à 14, muni d'un cathéter de perfusion (500) comprenant :
- une extrémité distale (502) destinée à être insérée dans un patient ;
- une extrémité proximale (504) destinée à être connectée avec une extrémité distale de la tubulure de la perfusion ;
- le dispositif de prélèvement (506-509) arrangé entre l'extrémité distale (502) et l'extrémité proximale (504), et comprenant une structure tubulaire (507) de connexion au circuit fluidique de la perfusion, munie :
∘ d'une zone d'intubation (Zi), en utilisation, d'un canal de prélèvement (300) comprenant une extrémité distale (301) ; et
∘ d'un moyen de maintien (503), en utilisation, dans la structure tubulaire, de la partie du canal de prélèvement (300) comprenant une extrémité distale (301), de sorte que, en utilisation, ladite extrémité distale (301) soit dirigée vers le cathéter de perfusion (101), dans le sens d'écoulement (F1) du produit de perfusion (102), depuis un réservoir (101) de produit de perfusion vers le cathéter de perfusion (101).

16. Système de perfusion sécurisé selon l'une quelconque des revendications 6 à 14, comprenant une tubulure de perfusion comprenant :
- une extrémité proximale (602) destinée à être connectée à un réservoir d'un produit de perfusion ;
- une extrémité distale (604) destinée à être connectée avec une extrémité proximale d'un cathéter de perfusion insérée dans un patient ;
- le dispositif de prélèvement (606-609) arrangé entre l'extrémité distale (602) et l'extrémité proximale (604), et comprenant une structure tubulaire (607) de connexion au circuit fluidique de la perfusion, munie :
∘ d'une zone d'intubation (Zi), en utilisation, d'un canal de prélèvement (300) comprenant une extrémité distale (301) ; et
∘ d'un moyen de maintien (603), en utilisation, dans la structure tubulaire, de la partie du canal de prélèvement (300) comprenant une extrémité distale (301), de sorte que, en utilisation, ladite extrémité distale (301) soit dirigée vers le cathéter de perfusion (101), dans le sens d'écoulement (F1) du produit de perfusion, depuis un réservoir (103) de produit de perfusion vers le cathéter de perfusion (101).

17. Système de perfusion sécurisé selon l'une des revendications 15 ou 16, dans lequel la zone d'intubation est disposée à une distance verticale maximale déterminée (Dvₘₐₓ) de l'extrémité proximale du cathéter.

18. Système de perfusion sécurisé selon la revendication 17, dans lequel la distance verticale maximale déterminée (Dvₘₐₓ) est comprise entre 0 cm et 50 cm.

## Patentansprüche

1. System zur gesicherten Infusion einer Körperflüssigkeit, **dadurch gekennzeichnet, dass** es umfasst:
- einen Infusionsflüssigkeitskreislauf für eine Infusion (100, 1000), umfassend einen Infusionskatheter (101), eine Infusionsleitung (102, 1002) und ein Reservoir (103, 1003) für ein Infusionsprodukt, welches dazu bestimmt ist, bei einem Patienten (B) infundiert zu werden;
ein Entnahmemittel (200, 400) für eine Körperflüssigkeit eines Patienten,
- ein Entnahmereservoir (303, 3000, 4000), welches mit einem Mittel zur Fluidverbindung (300) mit dem Entnahmemittel (200, 400) versehen ist,
- ein Mittel zur Analyse und zum Vergleich (700, 710, 720, 721) der entnommenen Körperflüssigkeit und einer Probe (810) des bestimmten Produkts,
- ein Mittel zur Fluidverbindung zwischen dem Mittel zur Analyse und dem Entnahmereservoir;
- ein Mittel zur Flusssteuerung (730, 731) des Infusionsprodukts in der Leitung, wobei das Mittel zur Analyse und zum Vergleich (700, 720) dazu ausgestaltet ist, an das Mittel zur Flusssteuerung (730, 731) eine Vergleichsinformation (I_{c}) der entnommenen Körperflüssigkeit (304) und der Probe des bestimmten Produkts (810) zu übertragen.

2. System zur gesicherten Infusion nach Anspruch 1, wobei das Mittel zur Analyse (700, 720) dazu ausgestaltet ist, das Mittel zur Flusssteuerung (730, 731) zu steuern, so dass es den Fluss des Infusionsprodukts blockiert, wenn die Vergleichsinformation (I_{c}) anzeigt, dass die Körperflüssigkeit und das Infusionsprodukt nicht kompatibel sind, und dass es den Durchfluss des Infusionsprodukt erlaubt, wenn die Vergleichsinformation (I_{c}) anzeigt, dass die Körperflüssigkeit und das Infusionsprodukt kompatibel sind.

3. System zur gesicherten Infusion nach einem der Ansprüche 1 oder 2, umfassend ein Mittel zur Anzeige (740, 741) der Vergleichsinformation (I_{c}) der entnommenen Körperflüssigkeit (304) und der Probe des bestimmten Produkts (810).

4. System zur gesicherten Infusion nach einem der Ansprüche 1 bis 3, wobei das Mittel zur Analyse (700, 720) dazu ausgestaltet ist, das Mittel zur Flusssteuerung (730, 731) zu steuern, so dass es automatisch den Fluss des Infusionsprodukts erzeugt, wenn die Körperflüssigkeit und das Infusionsprodukt kompatibel sind, und dass es den Fluss des Infusionsprodukts nicht erzeugt, wenn die Körperflüssigkeit und das Infusionsprodukt nicht kompatibel sind.

5. System zur gesicherten Infusion nach einem der Ansprüche 1 bis 4, wobei das Mittel zur Analyse (700) eine Reaktionskammer (710) und ein Erfassungsmittel (720, 721) umfasst.

6. System zur gesicherten Infusion nach einem der Ansprüche 1 bis 5, wobei das Entnahmemittel umfasst:
- einen Entnahmekanal (300, 2100), umfassend ein proximales Ende (302, 2003), welches dazu bestimmt ist, mit dem Entnahmereservoir (303) verbunden zu werden, und ein distales Ende (301, 2101), und
- eine Entnahmevorrichtung (400, 450, 500, 600, 2000), welche dazu bestimmt ist, in den Infusionsfluidkreislauf (100-1000) eingefügt zu werden und eine röhrenförmige Struktur (402, 452, 507, 607, 2002) zur Verbindung mit dem Infusionsfluidkreislauf umfasst, versehen mit:
• einer Zone zur Intubation (Zi), im Einsatz, eines Entnahmekanals (300, 2100), welcher ein distales Ende (301, 2101) umfasst; und
• ein Mittel zum Halten (403, 408, 454, 503, 510, 603, 610, 2004, 2004a), im Einsatz, in der röhrenförmigen Struktur, des Teils des Entnahmekanals (300, 2100), welcher ein distales Ende (301, 2101) umfasst, so dass, im Einsatz, das distale Ende (301, 2101) in Richtung des Infusionskatheters (101), in der Flussrichtung (F1) des Infusionsprodukts (102), von einem Reservoir (103) für das Infusionsprodukt in Richtung des Infusionskatheters (101) geführt wird.

7. System zur gesicherten Infusion nach Anspruch 6, wobei die Zone zur Intubation so eingerichtet ist, dass sie im Einsatz in einer bestimmten maximalen Distanz (Dvₘₐₓ) von dem proximalen Ende des Katheters angeordnet ist.

8. System zur gesicherten Infusion nach Anspruch 7, wobei die bestimmte maximale vertikale Distanz (Dvₘₐₓ) zwischen 0 cm und 50 cm enthalten ist.

9. System zur gesicherten Infusion nach einem beliebigen der Ansprüche 6 bis 8, wobei der das distale Ende (1101) umfassende Teil des Entnahmekanals (1100) in der röhrenförmigen Struktur (1002) befestigt ist und ein ein proximales Ende umfassender Teil (1003) des Entnahmekanals (1100) jenseits der röhrenförmigen Struktur (1002) auf Höhe der Zone zur Intubation (2i) hervortritt.

10. System zur gesicherten Infusion nach einem der Ansprüche 6 bis 8, wobei die Zone zur Intubation ein Einsetzmittel (408, 454 ,508, 510, 608, 610) umfasst, welches dazu ausgestaltet ist, im Einsatz das Einsetzen des das distale Ende (301) umfassenden Teils des Entnahmekanals (300) in der röhrenförmigen Struktur zu erlauben.

11. System zur gesicherten Infusion nach Anspruch 10, wobei das Einsetzmittel ausgewählt ist aus:
- eine Membran (408, 454, 508, 510, 608, 610) aus einem dichten Material, welches seine Dichtigkeit beibehält, nachdem es durchstochen wurde; und
- ein dichter Verbinder.

12. System zur gesicherten Infusion nach Anspruch 11, wobei das dichte Material, welches seine Dichtigkeit beibehält, nachdem es durchstochen wurde, ausgewählt ist aus einem Silikonpolymer, wie z.B. Polydimethylsiloxan (PDMS), Polymethylmethacrylat (PMMA) und Polyvinylchlorid (PVC).

13. System zur gesicherten Infusion nach einem der Ansprüche 6 bis 12, wobei die Entnahmevorrichtung außerdem umfasst:
- ein Mittel zur Verbindung mit einem proximalen Ende des Infusionskatheters, und
- ein Mittel zur Verbindung mit einem distalen Ende der Infusionsleitung.

14. System zur gesicherten Infusion nach einem der Ansprüche 6 bis 13, wobei der Entnahmekanal (300) ein Länge zwischen seinen zwei Enden aufweist, welche zwischen 10 cm und 100 cm, bevorzugt zwischen 20 cm und 50 cm enthalten ist.

15. System zur gesicherten Infusion nach einem der Ansprüche 6 bis 14, versehen mit einem Infusionskatheter (500), welcher umfasst:
- ein distales Ende (502), welches dazu bestimmt ist, in einen Patienten eingesetzt zu werden;
- ein proximales Ende (504), welches dazu bestimmt ist, mit einem distalen Ende der Infusionsleitung verbunden zu werden;
- die Entnahmevorrichtung (506-509), welche zwischen dem distalen Ende (502) und dem proximalen Ende (504) angeordnet ist, und eine röhrenförmige Struktur (507) zur Verbindung mit dem Infusionsfluidkreislauf umfasst, versehen mit:
• einer Zone zur Intubation (Zi), im Einsatz, eines Entnahmekanals (300), welcher ein distales Ende (301) umfasst; und
• ein Mittel zum Halten (503), im Einsatz, in der röhrenförmigen Struktur, des ein distales Ende (301) umfassenden Teils des Entnahmekanals (300), so dass, im Einsatz, das distale Ende (301) in Richtung des Infusionskatheters (101), in der Flussrichtung (F1) des Infusionsprodukts (102), von einem Reservoir (101) für das Infusionsprodukt in Richtung des Infusionskatheters (101) geführt wird.

16. System zur gesicherten Infusion nach einem der Ansprüche 6 bis 14, umfassend eine Infusionsleitung, welche umfasst:
- ein proximales Ende (602), welches dazu bestimmt ist, mit einem Reservoir für ein Infusionsprodukt verbunden zu werden;
- ein distales Ende (604), welches dazu bestimmt ist, mit einem proximalen Ende eines in einen Patienten eingesetzten Infusionskatheters verbunden zu werden;
- die Entnahmevorrichtung (606-609), welche zwischen dem distalen Ende (602) und dem proximalen Ende (604) angeordnet ist und eine röhrenartige Struktur (607) zur Verbindung mit dem Infusionsfluidkreislauf umfasst, versehen mit:
• einer Zone zur Intubation (Zi), im Einsatz, eines Entnahmekanals (300), welcher ein distales Ende (301) umfasst; und
• ein Mittel zum Halten (603), im Einsatz, in der röhrenförmigen Struktur, des ein distales Ende (301) umfassenden Teils des Entnahmekanals (300), so dass, im Einsatz, das distale Ende (301) in Richtung des Infusionskatheters (101), in der Flussrichtung (F1) des Infusionsprodukts (102), von einem Reservoir (103) für das Infusionsprodukt in Richtung des Infusionskatheters (101) geführt wird.

17. System zur gesicherten Infusion nach einem der Ansprüche 15 oder 16, wobei die Zone zur Intubation mit einer bestimmten maximalen vertikalen Distanz (Dvₘₐₓ) zu dem proximalen Ende des Katheters angeordnet ist.

18. System zur gesicherten Infusion nach Anspruch 17, wobei die bestimmte maximale vertikale Distanz (Dvₘₐₓ) zwischen 0 cm und 50 cm enthalten ist.

## Claims

1. A system for the secure perfusion of a body fluid, **characterized in that** it comprises:
- a fluid circuit of a perfusion (100, 1000) comprising a perfusion catheter (101), perfusion tubing (102, 1002) and a container (103, 1003) of given perfusion product to be perfused to a patient (B);
- a means (200, 400) for collecting a sample of a body fluid from a patient;
- a sample collection container (303, 3000, 4000) equipped with a means (300) of fluid connection with the sample collection means (200, 400);
- a means (700, 710, 720, 721) of analyzing and comparing the collected body fluid and a sample (810) of given product;
- a means of fluid connection between the analysis means and the sample collection container;
- a means (730, 731) for controlling the flow of the perfusion product in the tubing, the analysis and comparison means (700, 720) being capable of transmitting to the flow control means (730, 731), a comparison information (I_{c}) of the collected body fluid (304) and the sample of given product (810).

2. The secure perfusion system as claimed in claim 1, wherein the analysis means (700, 720) is capable of controlling the flow control means (730, 731) so that it blocks the flow of the perfusion product if the comparison information (I_{c}) indicates that the body fluid and the perfusion product are incompatible, and so that it permits the flow of the perfusion product if the comparison information (I_{c}) indicates that the body fluid and the perfusion product are compatible.

3. The secure perfusion system as claimed in either one of claims 1 and 2, comprising a means (740, 741) for displaying the comparison information (I_{c}) of the body fluid collected (304) and the sample of given product (810).

4. The secure perfusion system as claimed in any one of claims 1 to 3, wherein the analysis means (700, 720) is capable of controlling the flow control means (730, 731) so that it automatically generates the flow of the perfusion product if the body fluid and the perfusion product are compatible, and so that it does not generate the flow of the perfusion product if the body fluid and the perfusion product are incompatible.

5. The secure perfusion system as claimed in any one of claims 1 to 4, wherein the analysis means (700) comprises a reaction chamber (710) and a detection means (720, 721).

6. The secure perfusion system as claimed in any one of claims 1 to 5, wherein the sample collection means comprises:
- a sample collection channel (300, 2100) comprising a proximal end (302, 2003) intended to be connected to the sample collection container (303), and a distal end (301, 2101), and
- a sample collection device (400, 450, 500, 600, 2000) intended to be incorporated into the fluid circuit of a perfusion (100, 1000), and comprising a tubular structure (402, 452, 507, 607, 2002) for connection to the fluid circuit of the perfusion, equipped:
∘ with a zone (Zi) of intubation, in use, of a sample collection channel (300, 2100) comprising a distal end (301, 2101); and
∘ with a means (403, 408, 454, 503, 510, 603, 610, 2004, 2004a) for holding, in use, in the tubular structure, the portion of the sample collection channel (300, 2100) comprising a distal end (301, 2101), so that, in use, said distal end (301, 2101) is pointed toward the perfusion catheter (101), in the flow direction (F1) of the perfusion product (102), from a container (103) of perfusion product to the perfusion catheter (101).

7. The secure perfusion system as claimed in claim 6, wherein the intubation zone is arranged so that, in use, it is positioned at a given maximum vertical distance (Dvₘₐₓ) from the proximal end of the catheter.

8. The secure perfusion system as claimed in claim 7, wherein the given maximum vertical distance (Dvₘₐₓ) is between 0 cm and 50 cm.

9. The secure perfusion system as claimed in any one of claims 6 to 8, wherein the portion comprising the distal end (2101) of the sample collection channel (2100) is fixed in the tubular structure (2002), and a portion (2003) comprising a proximal end of the sample collection channel (2100) emerges outside of the tubular structure (2002) level with the intubation zone (Zi).

10. The secure perfusion system as claimed in any one of claims 6 to 8, wherein the intubation zone comprises an insertion means (408, 454, 508, 510, 608, 610) suitable for enabling, in use, the insertion, into the tubular structure, of the portion of the sample collection channel (300) comprising the distal end (301).

11. The secure perfusion system as claimed in claim 10, wherein the insertion means is chosen from:
- a membrane (408, 454, 508, 510, 608, 610) made of a leaktight material that retains its leaktightness after having been pierced; and
- a leaktight connector.

12. The secure perfusion system as claimed in claim 11, wherein the leaktight material that retains its leaktightness after having been pierced is chosen from a silicone polymer, such as polydimethylsiloxane (PDMS), polymethyl methacrylate (PMMA), and polyvinyl chloride (PVC).

13. The secure perfusion system as claimed in any one of claims 6 to 12, wherein the sample collection device additionally comprises:
- a means of connection to a proximal end of the perfusion catheter, and
- a means of connection to a distal end of the perfusion tubing.

14. The secure perfusion system as claimed in any one of claims 6 to 13, wherein the sample collection channel (300) has a length between its two ends of between 10 cm and 100 cm, prefierably between 20 cm and 50 cm.

15. The secure perfusion system as claimed in any one of claims 6 to 14, equipped with a perfusion catheter (500) comprising:
- a distal end (502) intended to be inserted into a patient;
- a proximal end (504) intended to be connected with a distal end of the perfusion tubing;
- the sample collection device (506-509) arranged between the distal end (502) and the proximal end (504), and comprising a tubular structure (507) for connection to the fluid circuit of the perfusion, equipped:
o with a zone (Zi) of intubation, in use, of a sample collection channel (300) comprising a distal end (301); and
o with a means (503) for holding, in use, in the tubular structure, the portion of the sample collection channel (300) comprising a distal end (301), so that, in use, said distal end (301) is pointed toward the perfusion catheter (101), in the flow direction (F1) of the perfusion product (102), from a container (103) of perfusion product to the perfusion catheter (101).

16. The secure perfusion system as claimed in any one of claims 6 to 14, comprising perfusion tubing comprising:
- a proximal end (602) intended to be connected to a container of a perfusion product;
- a distal end (604) intended to be connected with a proximal end of a perfusion catheter inserted into a patient;
- the sample collection device (606-609) arranged between the distal end (602) and the proximal end (604), and comprising a tubular structure (607) for connection to the fluid circuit of the perfusion., equipped:
o with a zone (Zi) of intubation, in use, of a sample collection channel (300) comprising a distal end (301); and
o with a means (603) for holding, in use, in the tubular structure, the portion of the sample collection channel (300) comprising a distal end (301), so that, in use, said distal end (301) is pointed toward the perfusion catheter (101), in the flow direction (F1) of the perfusion product, from a container (103) of perfusion product to the perfusion catheter (101).

17. The secure perfusion system as claimed in either of claims 15 and 16, wherein the intubation zone is positioned at a given maximum vertical distance (Dvₘₐₓ) from the Pproximal end of the catheter.

18. The secure perfusion system as claimed in claim 17, wherein the given maximum vertical distance (Dvₘₐₓ) is between 0 cm and 50 cm.
